# EUROPEAN PATENT SPECIFICATION

(11) **EP 1 957 666 B1**
(45) Date of publication and mention of the grant of the patent: **05.11.2014**
(21) Application number: 06847293.5
(22) Date of filing: 08.12.2006
(51) Int. Cl.: C12Q 1/18, C12Q 1/48, G01N 33/50, G01N 33/569

(54) **USE OF 4'-PHOSPHOPANTETHEINYL TRANSFERASE AS A TARGET FOR IDENTIFYING ANTIBIOTIC MOLECULES**
VERWENDUNG EINER 4'-PHOSPHOPANTETHEINYL-TRANSFERASE ALS ZIEL BEI DER IDENTIFIZIERUNG ANTIBIOTISCHER MOLEKÜLE
UTILISATION DE 4'-PHOSPHOPANTETHEINYL TRANSFERASE COMME CIBLE POUR IDENTIFIER DES MOLECULES ANTIBIOTIQUES

(30) Priority: 08.12.2005 EP 05292610
(43) Date of publication of application: 20.08.2008
(73) Proprietor: Centre National de la Recherche Scientifique, 75016 Paris (FR); Université Paul Sabatier (Toulouse III), 31062 Toulouse Cedex 9 (FR)
(72) Inventor: CHALUT, Christian, F-31860 Labarthe Sur Leze (FR); GUILHOT, Christophe, F-31180 Castelmaurou (FR)
(74) Representative: Marcadé, Véronique
(86) International application number: PCT/IB2006/004075
(87) International publication number: WO 2007/069089

(56) References cited:
- WO-A-2005/024007
- US-B1- 6 579 695
- US-B1- 6 951 729
- FERRERAS JULIAN A ET AL: "Small-molecule inhibition of siderophore biosynthesis in Mycobacterium tuberculosis and Yersinia pestis." NATURE CHEMICAL BIOLOGY. JUN 2005, vol. 1, no. 1, June 2005 (2005-06), pages 29-32, XP002372784 ISSN: 1552-4450
- QUADRI L E ET AL: "Identification of a Mycobacterium tuberculosis gene cluster encoding the biosynthetic enzymes for assembly of the virulence-conferring siderophore mycobactin." CHEMISTRY & BIOLOGY. NOV 1998, vol. 5, no. 11, November 1998 (1998-11), pages 631-645, XP000979840 ISSN: 1074-5521 cited in the application
- VEYRON-CHURLET ROMAIN ET AL: "The biosynthesis of mycolic acids in Mycobacterium tuberculosis relies on multiple specialized elongation complexes interconnected by specific protein-protein interactions." JOURNAL OF MOLECULAR BIOLOGY. 4 NOV 2005, vol. 353, no. 4, 4 November 2005 (2005-11-04), pages 847-858, XP005118795 ISSN: 0022-2836
- BOBROV ALEXANDER G ET AL: "Yersiniabactin production requires the thioesterase domain of HMWP2 and YbtD, a putative phosphopantetheinylate transferase", INFECTION AND IMMUNITY, vol. 70, no. 8, August 2002 (2002-08), pages 4204-4214, ISSN: 0019-9567
- LUKE R K J ET AL: "LOCATION OF 3 GENES CONCERNED WITH THE CONVERSION OF 2 3 DI HYDROXY BENZOATE INTO ENTEROCHELIN IN ESCHERICHIA-COLI K-12", JOURNAL OF BACTERIOLOGY, vol. 107, no. 2, 1971, pages 557-562, ISSN: 0021-9193
- Leonard Karel and Elizabeth Spencer Roach: "A dictionary of antibiotics", 1 January 1951 (1951-01-01), Columbia University Press, New York pages 1-1,
- IAN CHOPRA: "Antibiotics", 21 August 2001 (2001-08-21), John Wiley & Sons, online pages 1-9,
- LEBLANC CÉCILE ET AL: "4'-Phosphopantetheinyl transferase PptT, a new drug target required for Mycobacterium tuberculosis growth and persistence in vivo.", PLOS PATHOGENS DEC 2012, vol. 8, no. 12, December 2012 (2012-12), page e1003097, ISSN: 1553-7374

## Description

The cell envelope plays a major role in the physiology of *Mycobacterium tuberculosis,* the causative agent of tuberculosis in human, a disease still responsible for more death than any other single infectious agent. First, this complex structure provides a strong resistance to degradation by host enzymes and a permeability barrier to antibiotics and to toxic molecules produced by the host. Second, it contains components which exert an active effect to facilitate uptake of the bacterium and to modulate the host immune response (Daffé and Draper, 1998). The mycobacterial envelope is characterized by a very high lipid content (60 % of the dry weight) and the occurrence of lipids with unusual structures (Daffé and Draper, 1998). The major lipid constituents of this cell wall are the mycolic acids. These molecules are specifically found in the *Corynebacterineae* suborder including corynebacteria, mycobacteria, nocardia or rhodoccoci where they exist either as esters of trehalose or esterified to the arabinogalactan core of the bacteria cell wall (Daffé and Draper, 1998; Daffé, 2005). All mycolic acids consist of α-alkyl β-hydroxy branched fatty acids but their size and their structure differ according to the bacterial species (Asselineau et al., 2002). For instance mycobacterial mycolic acids are made of very long carbon chains (C₆₀-C₉₀) containing additional motifs such as oxygen functions, cyclopropyl ring or methyl branches whereas *Corynebacteria* spp produce a mixture of saturated and insaturated corynomycolic acids which typically range in size from 30 to 36 carbons. Mycolic acids are key structural components of the cell envelope and their biosynthesis pathway is the target of the major antituberculous drug, isoniazid (Banerjee et al., 1994). The structure of mycolate has been found to be critical for initial replication and persistence *in vivo* (Takayama et al., 2005). In slow growing mycobacteria, mycolic acids are associated with a number of extractable lipids containing methyl-branched fatty acids (Minnikin et al., 2002). Various studies have shown that some of these compounds, such as phthiocerol dimycocerosates (DIM) and phenolic glycolipid (PGL-tb), contribute to pathogenicity of *M. tuberculosis* (Camacho et al., 1999; Reed et al., 2004).

The biosynthesis of the various and unusual lipids of *M. tuberculosis* involves the combined action of fatty acid synthase (Fas) systems and type-I polyketide synthases (Pks). For instance, the formation of mycolates required two Fas systems and one Pks: Fas-I, a multifunctional protein, is dedicated to the production of short C_{16,18} fatty acids; Fas-II, a complex of monofunctional proteins elongates fatty acids generated by Fas-I to yield long chain fatty acids ranging from C₄₈ to C₆₄ in length; Pks13 catalyses the condensation of two fatty acids to form mycolic acids (Takayama et al., 2005; Portevin et al.; 2004). Biosynthesis of DIM and PGL-tb requires Fas-I and seven Pks (Onwueme et al., 2005). Finally, Fas-I and Pks2 or Pks3/4 are involved in the formation of the various multimethyl-branched fatty acids found in the trehalose derived lipids specific of *M*. *tuberculosis* (Sirakova et al., 2001; Dubey et al., 2002). Overall, the genome of *M. tuberculosis* encodes more than eighteen type-I Pks and two Fas systems (Cole et al., 1998). These enzymes are the key players which endow *M. tuberculosis* with the unique ability to produce an impressive variety of lipids of unique structure.

To be functional, the acyl carrier protein (ACP) domains of Fas and Pks need to be converted from their inactive apo-forms to their functional holo-forms by the covalent attachment of a 4'-phosphopantetheine (P-pant) group to an hydroxyl group of an invariant serine residue (Walsh et al., 1997; Keating and Walsh, 1999). The role of this flexible prosthetic arm is to provide an attachment site for chain-extension intermediates and to shuttle the growing chains between the different catalytic sites of the synthase complexes (Cane and Walsh, 1999). This feature is shared by another class of enzymes, the non-ribosomal peptide synthases (NRPS), which are involved in the production of siderophores in *M*. *tuberculosis* (De Voss et al., 1999). This posttranslational modification is catalyzed by an 4'-phosphopantetheinyl transferase (PPTase) which transfers the P-pant group from coenzyme A (CoA) to the ACP (Lambalot et al., 1996). PPTases have been identified and biochemically characterized in a number of microorganisms and have been classified in three groups based on primary sequence similarity and substrate specificity (Wiessman et al., 2004 and references therein). Members of the first group exemplified by the Holo-(acyl carrier protein) synthase (AcpS) of *Escherichia coli,* are about 120 residues in size and act as homotrimers (Parris et al., 2000). These AcpS-type PPTases have a narrow substrate specificity limited to the ACP of type-II Fas and Pks systems (Mootz et al., 2001). The second group comprises PPTases ressembling the Sfp (Surfactin phosphopantetheinyl transferase) protein, a PPTase required for the production of the antibiotic surfactin in *Bacillus subtilis* (Quadri et al., 1998a). These Sfp-type PPTases are about twice the size than those of the first group (220-240 residues) and exist under monomeric form (Mofid et al., 2004). They are often associated with genes encoding the production of secondary metabolites but exhibit very broad substrate specificities and are usually able to modifying both type-I and type-II ACP and peptidyl carrier protein (PCP) domains (Weissman et al., 2004; Quadri et al., 1998a). PPTases of the third group are incorporated as a catalytic domain in type-I Fas and allow self-phosphopantetheinylation of the ACP domain of the protein (Fichtlscherer et al., 2000).

Usually bacteria contain more than one PPTase dedicated to one or several P-pant dependant pathways. For instance, *E. coli* has three PPTases (Lambalot et al., 1996), AcpS involved in fatty acid synthesis, EntD a Sfp-type PPTase involved in the biosynthesis of the siderophore enterobactin and the product of gene *yhhU* which has an unknown physiological function. It has been shown that AcpS is essential for cell viability but not EntD (Flugel et al., 2000). In contrast, in *Bacillus subtilis* which has two PPTases, Sfp can complement the activity of AcpS and sustain fatty acids biosynthesis after inactivation of *acpS* (Mootz et al., 2001).

In their initial analysis of *M. tuberculosis* genome, Cole et al. (1998) identified *Rv2523c* as a gene encoding a putative PPTase related to AcpS. At the same time, a second PPTase was discovered in *M. tuberculosis* by Quadri *et al* (1998b) who found that *Rv2794c* encodes a Sfp-type PPTase, renamed PptT, that was able to activate *in vitro,* two NRPS required for the assembly of siderophore mycobactin. However, in spite of the importance of the Fas-I system and type-I Pks for the biology of *M*. *tuberculosis,* no data concerning the posttranslational modification of these enzymes have been reported in mycobacteria. Two PPTases have been identified which would be responsible for the activation of more than 20 proteins in *M. tuberculosis* but their respective repertoire of substrates, their putative redundancy and their importance for the mycobacterial biology have not been previously investigated. Answering these questions is crucial to understand the biosynthesis of lipids in *M*. *tuberculosis.*

The inventors have now found that orthologs of the two previously identified *M. tuberculosis* PPTases are found in other *Corynebacterineae* species. They demonstrated that both PPTases are essential for growth of mycobacteria and display identical functions in mycobacteria and corynebacteria: AcpS is responsible for the posttranslational modification of Fas-I and PptT, the Sfp-type PPTase, is involved in the activation of the condensing enzyme Pks13. In addition, they showed that the various type-I Pks required for the formation of lipid virulence factors are activated by PptT in *M. tuberculosis.* The involvement of PptT of *M. tuberculosis* in several P-pant dependent pathways essential for growth or virulence makes it an attractive potential target for the development of new antimycobacterial drugs.

A first aspect of the present invention is hence the use of a PptT protein according to claim 1 as a target for screening compounds for identifying those having an antibiotic activity. According to this invention, said PptT protein is preferably from a pathogenic bacterium containing mycolic acids, and more preferably a corynebacterium, a mycobacterium, or a nocardia. For example, the PptT protein is from a bacterium selected in the group consisting of *Corynebacterium diphtheriae, Corynebacterium minutissimum, Corynebacterium pseudotuberculosis, Mycobacterium africanum, Mycobacterium avium, Mycobacterium chelonae, Mycobacterium fortuitum, Mycobacterium kansasii, Mycobacterium leprae, Mycobacterium malmoense, Mycobacterium marinum, Mycobacterium microti, Mycobacterium paratuberculosis, Mycobacterium scrofulaceum, Mycobacterium simiae, Mycobacterium szulgai, Mycobacterium tuberculosis, Mycobacterium ulcerans, Mycobacterium xenopi, Nocardia asteroids, Nocardia brasiliensis, Nocardia farcinica, Nocardia nova, and Nocardia otitidiscaviarum.* In particular, PptT proteins of SEQ Nos: 2 to 9 can be used according to the invention.

Of course, fusion proteins corresponding to a PptT protein as described above, fused to a peptidic moiety that enables its easier purification without modifying its activity, are herein also considered as PptT proteins.

The present invention also pertains to an *in vitro* screening process according to claim 5 for identifying compounds having an antibiotic activity, by measuring the activity of a PptT protein in the presence or absence of said compounds.

In a process according to the invention, the PptT activity can be measured by (i) incubating an isolated PptT with a Pks protein or subunit thereof comprising an acyl carrier protein domain, and with a labelled Acetyl-CoA, (ii) precipitating said Pks protein or subunit thereof, and (iii) measuring the level of labelling of said precipitate. Any PptT protein as above-described can be used in such a test.

The present invention is illustrated by the experimental results and the figures described below.

### FIGURE LEGENDS

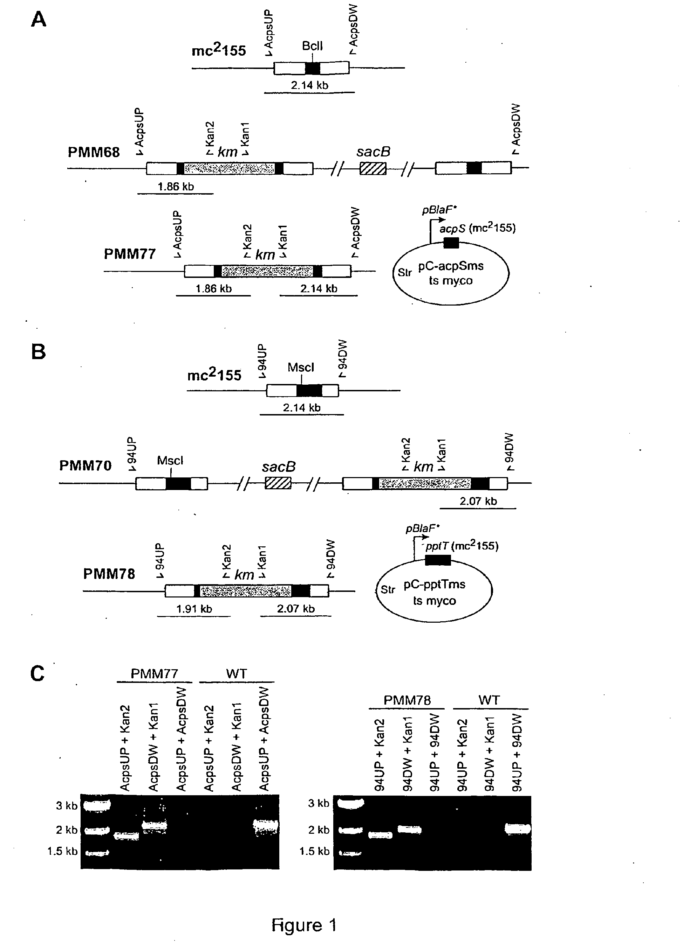

**Figure 1**: Construction of *M. smegmatis* mutants. Schematic representation of the genomic organisation of the *acpS* locus in the WT strain of *M*. *smegmatis,* the merodiploic PMM68 strain and the conditional PMM77 (*ΔacpS::km:p*C-acpSms) mutant (A) and of the *pptT* locus in the WT strain of *M. smegmatis*, the merodiploic PMM70 strain and the conditional PMM78 (*ΔpptT::km*:pC-pptTms) mutant (B). Black boxes represent the *acpS* (A) and the *pptT* (B) genes and white boxes represent the 5' and 3' flanking regions amplified by PCR to construct the mutants. The Km resistant cassette used for targeted disruption and the *sacB* gene are represented by gray and hatched boxes, respectively. Positions and names of primers (arrows) used for the characterization of mutants are shown and the expected sizes for PCR products are indicated below each genetic structure. (C) Recombinant strains PMM77 (left panel) and PMM78 (right panel) were analyzed by PCR using various combinaison of primers as indicated.

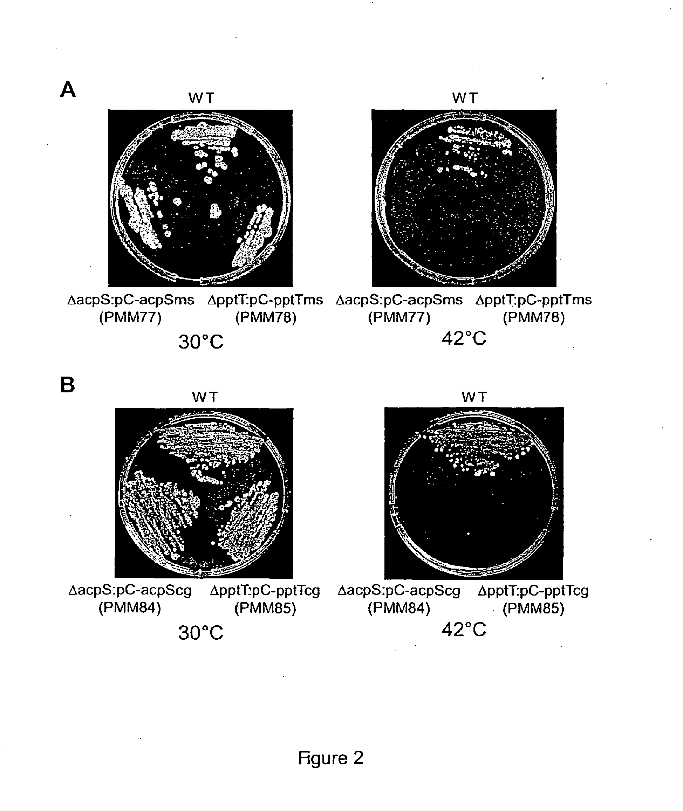

Figure 2 : Thermosensitivity of the *M. smegmatis* conditional mutants and genetic complementations with the *acpS* and *pptT* genes of C. *glutamicum. M. smegmatis* WT, PMM77 (*ΔacpS*:pC-acpSms) and PMM78 (*ΔpptT*:pC-pptTms) (A) and *M. smegmatis* WT, PMM84 (*ΔacpS*:pC-acpScg) and PMM85 (*ΔpptT*:pC-pptTcg) (B) were grown in LB (with Km and Str for the recombinant strains) at 30°C and streaked onto LB agar plates which were incubated for two days at either 30°C (left panels) or 42°C (right panels), a non permissive temperature for plasmid replication.

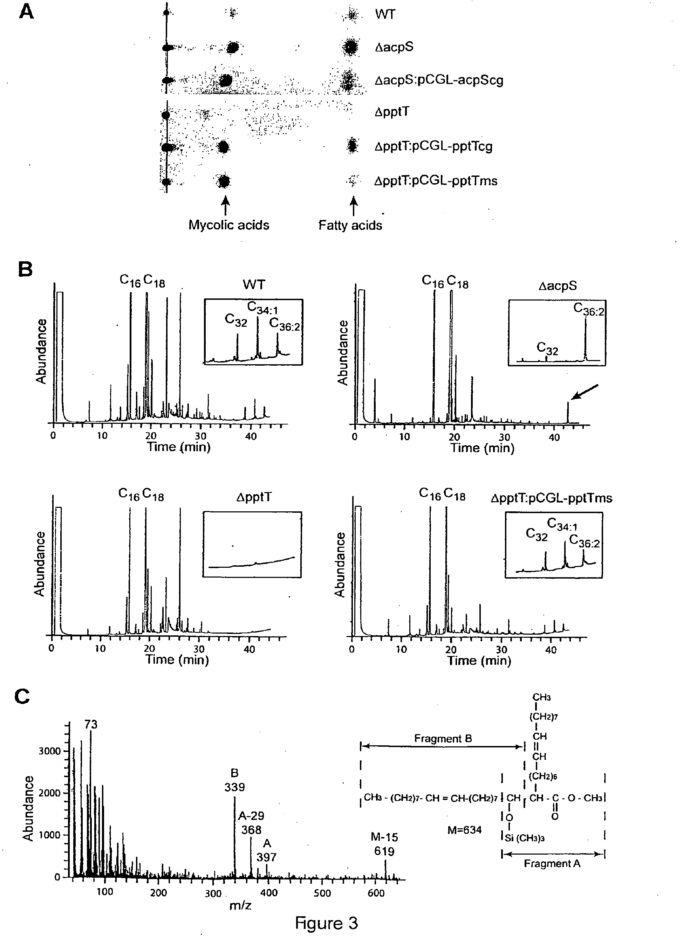

Figure 3 : Biochemical analysis of the cell envelope of *C*. *glutamicum Δacps::km* and *ΔpptT::km* recombinant strains. (A) Fatty acids and mycolic acids produced by C. *glutamicum* WT, the various mutants and the complemented strains were prepared from cells and separated by analytical TLC on Durasil 25TLC (Macherey-Nagel) with dichloromethane. Lipids were visualized by spraying the plates with 10% phosphomolybdic acid in ethanol followed by heating. (B) Trimethylsilyl derivatives of fatty acid methyl esters from C. *glutamicum* WT, *ΔacpS, ΔpptT*, *ΔpptT*:pCGL-pptTms were analysed by GC as described in Constant et al. (2002). For each chromatogram, the portion between 36 and 44 min is magnified. Peaks corresponding to C₁₆, C₁₈ fatty acids and C₃₂, C_{34:1}, C_{36:2} corynomycolates are indicated. (C) The trimethylsilyl derivative of C_{36:2} corynomycolate from *C*. *glutamicum ΔacpS* (shown by an arrow in B) was analyzed by GC-MS. The origin of the various major ion fragment peaks of the electron-impact mass spectrum are shown on the right.

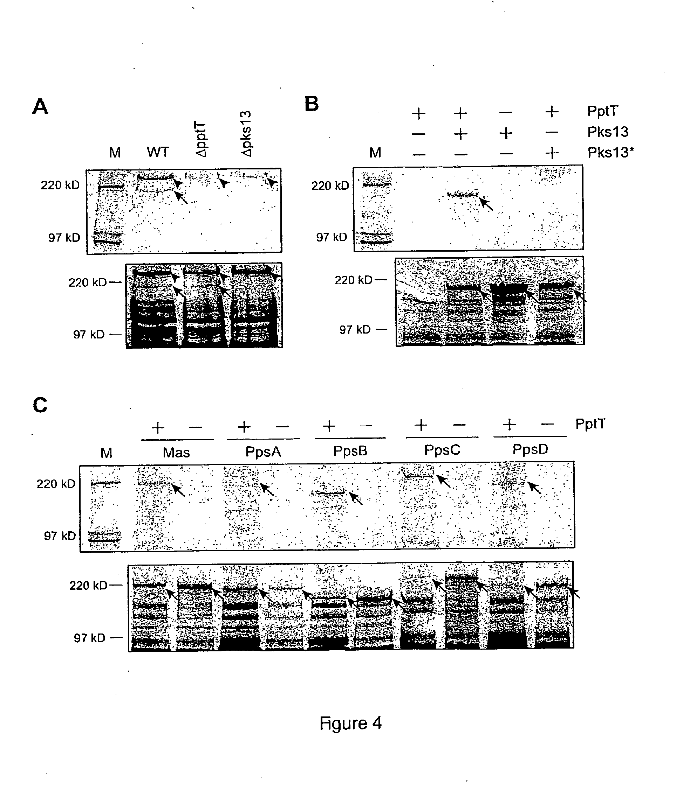

**Figure 4** **:** 4'-phosphopantetheinylation labelling assays using β-[β-¹⁴C]alanine. (A) C. *glutamicum* WT, *ΔpptT* and *Δpks13* were grown in presence of β-[β-¹⁴C]alanine and proteins from cellular extract of each strain were separated by SDS-PAGE. The polyacrylamide gel was stained with Coomassie blue (lower panel) and exposed to X-ray film for autoradiography (upper panel). Positions of Pks13 and Fas-I are indicated by arrows and arrow heads respectively. M, ¹⁴C-methylated markeurs (220, 97.4, 66, 46, 30, 21.5, 14.3 kD) (Sigma). (B-C) Strains of *E. coli* BL21 *ΔentD* expressing either Pks13 or the mutated Pks13* (Pks13, S55A and S1266A) from *M. tuberculosis* (B) or various type-I Pks from *M. tuberculosis* (C) with the wild type PptT (indicated by + on top of each figure) from *M. tuberculosis* or the mutated *PptT-*encoded PPTase (indicated by - on top of each figure) were grown in presence of β-[β-¹⁴C]alanine. After overexpression, cell extract proteins from each strain were separated by SDS-PAGE and polyacrylamide gels were stained with Coomassie blue [(B) and (C) lower panels] and exposed to X-ray film for autoradiography [(B) and (C) upper panels]. Positions of Pks13, Pks13* (B), Mas, PpsA, PpsB, PpsC and PpsD (C) are indicated by arrows. M, ¹⁴C-methylated markers.

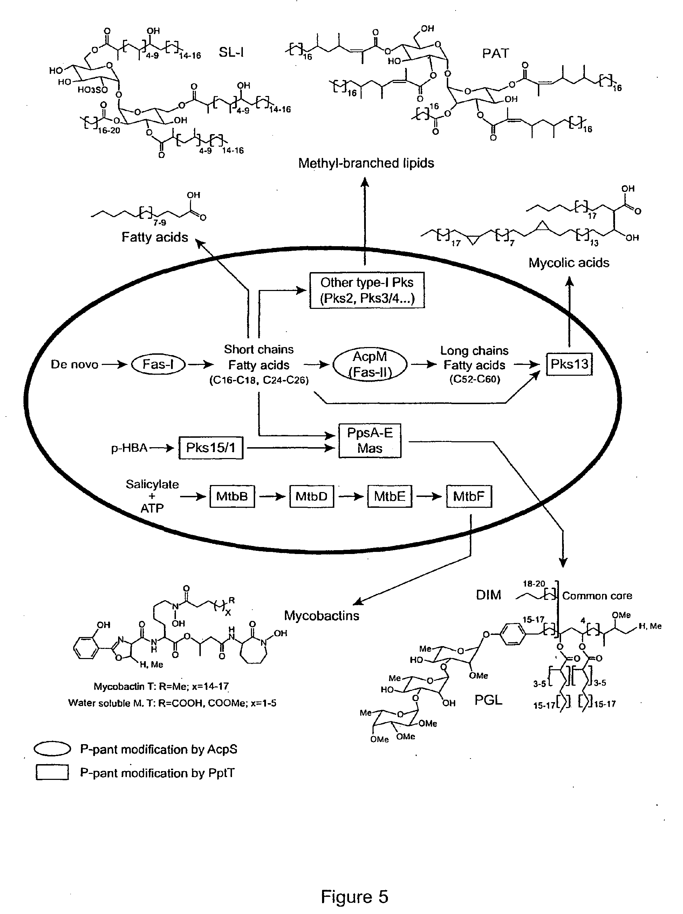

**Figure 5** **:** Schematic representation of the role played by AcpS and PptT in the fatty acids, mycolic acids, methyl-branched associated lipids and siderophores biosynthesis pathways in *M. tuberculosis.* Only proteins that require 4'-phosphopantetheinylation in these pathways are indicated. Proteins activated by AcpS are boxed by rectangles and proteins modified by AcpS by ovals. p-HBA, para-hydroxybenzoic acid, SL-1, sulfolipid 1, PAT, polyacyltrehaloses.

### EXAMPLES

### Example 1 : experimental procedures

### Bacterial strains and growth conditions

*E. coli* strains DH5α and C600 used for cloning experiments were grown in Luria Bertani (LB) Broth (Difco). *C. glutamicum* was cultured in Brain Heart Infusion (BHI) medium (Difco) containing 0.05 % of Tween 80 to prevent aggregation. The C. *glutamicum ΔacpS::km* mutant was grown in BHI medium supplemented with 0.03 % Tween 40 and 0.03 % (weight/volume) sodium oleate (Sigma). *M. smegmatis* was grown in LB Broth supplemented with 0.05% Tween 80. When required ampicillin (Amp), kanamycin (Km), chloramphenicol (Clm), streptomycin (Str), Hygromycin B (Hyg) and sucrose were used at a final concentration of 100 µg/ml, 40 µg/ml (for E. *coli*) or 25 µg/ml (for *M. smegmatis* and *C*. *glutamicum*), 15 µg/ml, 25 µg/ml, 200 µg/ml (for *E. coli*) or 50 µg/ml (for *M. smegmatis*) and 5 % (weight/volume), respectively.

### Construction of plasmids for the production of the recombinant PptT, Pks13, Mas and PpsA-D proteins of M. tuberculosis in E. coli.

The *pks13 (Rv3800), mas, ppsA, ppsB, ppsC* and *ppsD* genes were amplified by PCR from *M tuberculosis* H37Rv total DNA and the resulting fragments were inserted into the pET26b *E. coli* expression vector (Novagen) under the control of the *T7* promoter to give plasmids pWM35, pETMas, pETA, pETB, pETC, pETD, respectively. These vectors allow expression of recombinant Pks13, Mas and PpsA-D proteins fused to a polyhistidine tag peptide at their C-terminal ends in the BL21 *ΔentD E.coli* strain. Plasmid pWM35γ originates from pWM35 following site-directed mutagenesis of the two codons corresponding to the two catalytic residues Ser55 and Ser1266 responsible for the attachment of the P-pant moiety of CoA on the N- and C-terminal ACP domains of Pks13. In pWM35γ, these codons have been substituted by alanine encoding codons.

To coproduce PptT and the various Pks of *M. tuberculosis* in *E. coli, pptT (Rv2794c)* was amplified from *M. tuberculosis* H37Rv genomic DNA and inserted into plasmid pET26b downstream of the *T7* promoter. The *pptT* gene plus 108 bp upstream of the start codon, a region carrying the *T7* promotor region was reamplified by PCR and cloned into the *Bcl*I site of plasmid pLysS (Novagen) to give pLSfp. A derivative vector of pLSfp, named pLSfpΔ producing a non-functional truncated PptT protein was also made by digesting pLSfp by *Eco*RI which cut within the *pptT* gene. DNA ends were filled-in with Klenow fragment and the plasmid religated. All these constructs were checked by DNA sequencing (Genome Express, Grenoble, France).

The strains and plasmids used in the present study are summarized in Table 1 below.

**Table 1 : Strains or Plasmids**

| Name | Relevant characteristics | Ref./source |
|---|---|---|
| Strains | | |
| BL21(DE3) | *E. coli* | Novagen |
| BL21 *ΔentD* | *E. coli* BL21 (DE3)*ΔentD* | This study |
| mc²155 | *M*. *smegmatis* | Snapper et al., 1990 |
| PMM68 | *M. smegmatis* mc²155 harboring WT *acpS* and *ΔacpS::km* chromosomal alleles (first recombinaison event), Km^{R} | This study |
| PMM70 | *M. smegmatis* mc²155 harboring WT *pptT* and *ΔpptT::km* chromosomal alleles (first recombinaison event), Km^{R} | This study |
| PMM77 | *M. smegmatis* mc²155 *ΔacpS::km* carrying plasmid pC-acpSms, Km^{R} Str^{R} | This study |
| PMM78 | *M. smegmatis* mc²155 *ΔpptT::km* carrying plasmid pC-pptTms, Km^{R} Str^{R} | This study |
| PMM84 | *M. smegmatis* mc²155 *ΔacpS::km* carrying plasmid pC-acpScg, Km^{R} Str^{R} | This study |
| PMM85 | *M. smegmatis* mc²155 *ΔpptT::km* carrying plasmid pC-pptTcg, Km^{R} Str^{R} | This study |
| ATCC13032 | *C. glutamicum* | ATCC |
| CGL2035 | *C. glutamicum* ATCC13032 *ΔpptT(NCgl1905)::km,* Km^{R} | This study |
| CGL2039 | *C. glutamicum* ATCC13032 *ΔacpS(NCgl2405)::km,* Km^{R} | This study |
| | | |

| **Plasmids** | | |
|---|---|---|
| pET26b | *E. coli* expression vector containing the T7 promoter, Km^{R} | Novagen |
| pWM35 | pET26b containing *pks13* (*Rv3800*) of *M. tuberculosis* H37Rv, Km^{R} | This study |
| pWM35γ | pET26b containing a mutated *pks13* gene (S55A, S1266A) of *M. tuberculosis* H37Rv, Km^{R} | This study |
| pETMas | pET26b containing *mas* of *M. tuberculosis* H37Rv, Km^{R} | This study |
| pETA | pET26b containing *ppsA* of *M. tuberculosis* H37Rv, Km^{R} | This study |
| pETB | pET26b containing *ppsB* of *M. tuberculosis* H37Rv, Km^{R} | This study |
| pETC | pET26b containing *ppsC* of *M. tuberculosis* H37Rv, Km^{R} | This study |
| pETD | pET26b containing *ppsD* of *M*. *tuberculosis* H37Rv, Km^{R} | This study |
| pLysS | *E. coli* vector containing the *T7 lysoryme* gene, Clm^{R} | Novagen |
| pLSfp | pLysS containing *pptT (Rv2794c)* of *M. tuberculosis* H37Rv under the control of the *T7* promoter, Clm^{R} | This study |
| pLSfpΔ | pLys containing a mutated *ΔpptT* gene of *M. tuberculosis* H37Rv under the control of the *T7* promoter, Clm^{R} | This study |
| pMCS5 | Cloning vector, Amp^{R} | MoBiTec |
| pMCS5ΔacpS | pMCS5 containing *ΔacpS* (*NCgl2405*)*::km* of C. *glutamicum,* Amp^{R}, Km^{R} | This study |
| pMCS5ΔpptT | pMCS5 containing *ΔpptT (NCgl1905)::km* of *C*. *glutamicum*,, Amp^{R}, Km^{R} | This study |
| pCGL482 | *E. coli*/*C. glutamicum* shuttle plasmid, Clm^{R} | Peyret et al., 1993 |
| pCGL-acpScg | pCGL482 containing *acpS (NCgl2405)* of *C*. *glutamicum,* Clm^{R} | This study |
| pCGL-pptTcg | pCGL482 containing *pptT (NCgl1905)* of *C*. *glutamicum,* Clm^{R} | This study |
| pCGL-pptTms | pCGL482 *containing pptT of M*. *smegmatis,* Clm^{R} | This study |
| pJQ200 | Mycobacterial suicide plasmid containing *sacB,* Gm^{R}, Suc^{R} | Quandt and Hynes, 1993 |
| pJ2523S | pJQ200 containing *ΔacpS::km* of *M. smegmatis,* Km^{R}, Gm^{R}, Suc^{R} | This study |
| pJ2794S | pJQ200 containing *ΔpptT::km* of *M. smegmatis,* Km^{R}, Gm^{R}, Suc^{R} | This study |
| pCG76 | Thermosensitive replicative *E*. *coli*/mycobacteria shuttle plasmid, Str^{R} | Guilhot et al., 1994 |
| pC-acpSms | pCG76 containing *acpS* of *M. smegmatis,* Str^{R} | This study |
| pC-pptTms | pCG76 containing *pptT* of *M. smegmatis,* Str^{R} | This study |
| pC-acpScg | pCG76 containing *acpS* (*NCgl2405*) of *C*. *glutamicum,* Str^{R} | This study |
| pC-pptTcg | pCG76 containing *pptT* (*NCgl1905*) of *C*. *glutamicum,* Str^{R} | This study |

### Construction of the C. glutamicum ΔacpS::km and ΔpptT::km mutants and complementation vectors.

The *C*. *glutamicum ΔacpS::km* and *ΔpptT::km* mutants were generated as previously described (Portevin et al., 2004). Two DNA fragments overlapping the *acpS* gene (*NCgl2405*) at its 5' and 3' extremities were amplified by PCR from C. *glutamicum* strain ATCC13032 total DNA and inserted, flanking a Km resistance cassette, into the vector pMCS5 (MoBiTec, Göttingen, Germany) to give pMCS5ΔacpS. Similarly, two DNA fragments overlapping the *pptT* gene (*NCgl1905*) at its 3' and 5' extremities were amplified and cloned into plasmid pMCS5. The Km resistance cassette was inserted between these two fragments to yield plasmid pMCS5ΔpptT.

These two plasmids were transferred into *C*. *glutamicum* by electroporation and transformants were selected on plates containing either Km (for pMCS5ΔpptT) or Km, 0.03% sodium oleate (Sigma) and 0.03% Tween 40 (for pMCS5Δacps) (Sigma). Transformants in which allelic exchange had occurred between the WT chromosomal *acpS* or *pptT* gene and the mutated-borne alleles were characterized by PCR using various primers combination. Two recombinant strains, C. *glutamicum ΔacpS::km* and *C*. *glutamicum ΔpptT::km* named CGL2039 and CGL2035, respectively, were selected for further studies.

To construct the complementation plasmids, the *acpS* and *pptT* genes of C. *glutamicum* were amplified by PCR from C. *glutamicum* genomic DNA and the *pptT* gene of *M. smegmatis* was amplified by PCR from plasmid pC-pptTms. PCR products were inserted into a modified pCGL482 (Peyret et al., 1993) under the control of the *cpsB* promoter to give plasmids pCGL-acpScg, pCGL-pptTcg and pCGL-pptTms, respectively.

### Construction of the M. smegmatis ΔacpS::km and ΔpptT::km conditional mutants and complementation vectors.

The construction of the *M. smegmatis* conditional mutant strains were performed as previously described (Portevin et al., 2004). Two fragments containing the *acpS* gene (ortholog of *Rv2523c* of *M. tuberculosis* H37Rv) and the *pptT* gene (ortholog of *Rv2794c* of *M. tuberculosis* H37Rv) flanked by their 5'- and 3'- ends were amplified by PCR from chromosomal DNA of *M. smegmatis* mc²155 and cloned into pGEM-T (Promega) to yield pG2523S and pG2794S. Each plasmid was linearized with a restriction enzyme which cut at a unique site within either the *acpS* or the *pptT* (*Bcl*I for pG2523S and *Msc*I for pG2794S) and ligated with a Km resistance cassette to give plasmids pG2523SK and pG2764SK. The 4.76 kb *Nde*I*-Apa*I fragment of pG2523SK and the 4.44 kb *Nde*I*-Apa*I fragment of pG2794SK that contained the disrupted allele of *acpS* and *pptT*, respectively, were inserted between the *Sma*I and *Apa*I sites of pJQ200 (Quandt and Hymes, 1993), a mycobacterial suicide plasmid harboring the counterselectable marker *sacB* to yield pJ2523SK and pJ2794SK, respectively. These constructs were electrotransferred into *M. smegmatis* mc²155 and transformants were plated on LB plates supplemented with Km at 37°C. For each transformation, several transformants were selected and characterized by PCR using various primers. Two strains harbouring a pattern corresponding to the insertion of pJ2523SK and pJ2794SK in the chromosome as a result of single recombination event were named PMM68 and PMM70, respectively and used for further studies (Figure 1A and 1B).

For the construction of the thermosensitive complementation plasmid pC-acpSms, the *acpS* gene of *M. smegmatis* mc²155 was amplified by PCR and cloned into a derivative of the *E. coli*-mycobacteria shuttle plasmid pMIP12 (Le Dantec et al., 2001) harboring a *Nde*I site downstream of the *pBlaF** promoter. The resulting construct was then digested with *Pac*I and *Nhe*I and the 1.78 kb fragment overlapping the *pBlaF** promoter, the *acpS* gene and the terminator region of pMIP12 was subsequently cloned into the vector pCG76 (Guilhot et al., 1994) which harbours a thermosensitive mycobacterial replicon and a streptomycin resistance to give pC-acpSms. The *pptT* gene of *M. smegmatis,* the *acpS* gene of C. *glutamicum* (*NCgl2405*) and the *pptT* gene of *C.glutamicum* (*NCgl1905*) were PCR-amplified from genomic DNA. Each PCR product was digested with *Nde*I and *Spe*I and ligated independently between the *Nde*I and *Spe*I restriction sites of pC-acpSms to yield pC-pptTms, pC-acpScg and pC-pptTcg respectively.

Vectors pC-acpSms and pC-acpScg were introduced in strain PMM68 and pC-pptTms and pC-pptTcg in strain PMM70 by electrotransformation. Transformants were selected on LB plates containing Km and Str at 30°C. For each transformation, one colony was resuspended in liquid media and grown at 30°C before plating onto LB plates containing Km, Str and 5% sucrose at 30°C to induced the second crossover event at either the *acpS* chromosomal locus (for PMM68 transformed with pC-acpSms or pC-acpScg) or the *pptT* chromosomal locus (for PMM70 transformed with pC-pptTms or pC-pptTcg). For each construct, several clones were screened by PCR using various primers (Figure 1) after preparation of genomic DNA. Two strains named PMM77 (*ΔacpS::km*:pC-acpSms) and PMM84 (*ΔacpS::km*:pC-acpScg) in which the WT copy of *acpS* has been replaced by the mutated *acpS::km* allele and two strains named PMM78 (*ΔpptT::km*:pC-pptTms) and PMM85 (*ΔpptT::km*:pC-pptTcg) in which the WT copy of *pptT* has been replaced by the mutation *pptT::km* allele were selected.

### Biochemical characterization offatty acids of C. glutamicum strains

Cultures of *C*. *glutamicum* were grown to exponential growth and fatty acids were prepared from cells and separated by analytical TLC on Durasil 25 according to Laval *et al* (2001). For GC and GC-MS analyses, trimethylsilyl derivatives of fatty acids were obtained and analysed as described previously (Constant et al., 2002).

### Labeling of the 4'-phosphopantetheinylated proteins in C. glutamicum

The *C.glutamicum* WT, *ΔpptT::km* and *Δpks13::km* strains were grown to exponential growth phase in 5 ml of CGXII minimal medium (Keilhauer et al., 1993) supplemented with 0.05% Tween 80 at 30°C. 5 µl of β-[β-¹⁴C]alanine (49 mCi/mmol; Sigma) were then added to each culture and cells were incubated for an additional 12 hours. Bacterial cells were harvested by centrifugation, washed twice with Phosphate Buffered Saline (PBS) and resuspended in 500 µl of PBS. 500 µl of glass beads were added to each bacterial suspension and cells were disrupted by agitation for 3 min in a Mini BeadBeater. Cellular extracts were centrifuged at 12000 rpm for 10 min at 4°C and proteins of the supernatants were subjected to sodium dodecyl sulfate-polyacrylamide gel electrophoresis (SDS-PAGE). The polyacrylamide gel was stained with Coomassie blue, dried and the radiolabelled proteins were detected by exposure to Kodak X-Ray films.

### Labeling of the recombinant mycobacterial Pks in E. coli.

Expression vectors pWM35, pWM35γ, pETMas, pETA, pETB, pETC and pETD were cotransferred with either pLSfp or pLSfpΔ into *E. coli* BL21 *ΔentD.* For each transformation, one bacterial colony was inoculated in 2 ml LB medium supplemented with Km and Clm and incubated overnight at 37°C. 200 µl of the preculture was recovered and centrifugated for 2 min at 4000 rpm. Bacterial cells were then washed twice with 500 µl of M9 medium (Sambrook and Russell, 2001), diluted in 5 ml of M9 medium with Km and Clm and grown at 30°C. When the optical density at 600 nm reached 0.5, 5 µl of 1M isopropyl-β-D-thiogalactopyranoside (IPTG) and 5 µl of β-[β-¹⁴C]alanine were added to the culture and incubation was continued for 4 additional hours at 30°C. 1 ml of culture was then centrifuged, cells were washed with 1 ml of 50mM Tris-HCl pH8.0 and finally resuspended in 100 µl of 50mM Tris-HCl pH8.0 containing 0.1% triton X-100. For labeling analysis, 10 µl of cells suspension were incubated with 10 µl of 2X denaturing buffer (Sambrook and Russell, 2001) at 95°C for 5 min before separation of proteins by SDS-PAGE. Polyacrylamide gels were stained by Coomassie blue before drying and exposition to autoradiography.

### Example 2 : Mycobacteria and corynebacteria contain two conserved PPTases

*M. tuberculosis* produces a number of proteins which must be converted from their inactive apo-forms to their functional holo-forms by transfer of the P-pant moiety of CoA to a conserved serine on their ACPs or PCPs domains/or subunit. These include proteins AcpM and Fas-I involved in fatty acids biosynthesis but also a number of type-I Pks and NRPS proteins that are required for the biosynthesis of mycolic acids, cell wall associated lipids and siderophores. Two genes encoding proteins with similarities to PPTases were previously identified on the genome of *M. tuberculosis* H37Rv: *Rv2523c,* also called *acpS* because of the similarities of the encoded 130 amino acids protein with AcpS of various bacterial origin, and *Rv2794c,* which was previously shown to encode a Sfp-type PPTase of 227 amino acids, named PptT, responsible for the modification of a set of NRPS involved in the biosynthesis of mycobactins (Quadri et al., 1998b). In order to look for additional PPTase genes, we searched the *M. tuberculosis* H37Rv genome with conserved motifs of PPTases as probes (Weissman et al., 2004). No novel gene was identified suggesting that *M. tuberculosis* contains just two PPTases responsible for the activation of 20 protein substrates.

We then addressed the question of whether these two PPTase genes were conserved in the various mycobacterial species and in the closely related corynebacterial species. In all the analysed genome (including *M. tuberculosis* strains H37Rv and CDC151, *M. bovis, M. bovis* BCG, *M. leprae, M. avium, M. smegmatis, M. microti, M. marinum, C. glutamicum, C. diptheriae* and *C*. *efficiens*), we found orthologs of *acpS* and *pptT.* The levels of proteins similarities were more than 80% for the mycobacterial proteins and around 40% for the corynebacterial proteins with their *M. tuberculosis* counterparts.

Therefore these bioinformatic analyses showed that the related mycobacterial and corynebacterial species contain only two highly conserved PPTases with different catalytic properties. However, none of these two proteins had been characterized in mycobacteria or corynebacteria.

### Example 3 : AcpS and PptT PPTases are essential for the viability of Mycobacteria.

In mycobacteria, there is a large number of protein which needed to be activated for being functional: for instance, two Fas systems, at least three NRPS and eighteen type-I Pks in *M. tuberculosis.* However, no information was available both on the repertoire of protein substrates recognized by each PPTases and on the putative redundancy of these two proteins. As several of the proteins requiring activation are involved in biological processes essential for the mycobacterial viability, we first addressed the question whether any of the two PPTase genes can be disrupted in the model mycobacterial strain *M. smegmatis.* Two non replicative plasmids carrying the counterselectable marker *sacB* and a disrupted allele of either *acpS* or *pptT* of *M. smegmatis* were constructed and inserted into the chromosome by single crossover to give strains PMM68 and PMM70, respectively (Figure 1A and 1B). Plating cultures of strains PMM68 or PMM70 on medium containing sucrose 5% failed to select the second recombination event suggesting that both *acpS* and *pptT* are essential for mycobacteria viability. To firmly establish the essentiality, we produced two conditional *M. smegmatis* mutants. First, PMM68 cells were transformed with plasmid pC-acpSms, a thermosensitive mycobacterial vector carrying a intact copy of the *acpS* gene allele under the control of the *pBlaF** promoter. In this genetic context, selection on LB agar containing sucrose at 30°C produced colonies in which the second recombination event between the two chromosomal alleles had occurred (Figure 1A and 1C). One clone named PMM77 (*ΔacpS::km*:pC-acpSms) containing a disrupted *acpS::km* gene on the chromosome and a functional *acpS* gene in the thermosensitive plasmid pC-acpSms was chosen for further work. The same strategy was used to generate PMM78 (Δ*pptT::km*:pC-pptTms), a recombinant strain harbouring a mutated *pptT::km* chromosomal gene and an intact *pptT* allele in the thermosensitive plasmid pC-pptTms (Figure 1 Band 1C).

To investigate the role of *acpS* and *pptT* in mycobacterial growth, cultures of recombinant strains PMM77, PMM78 and the WT strain of *M. smegmatis* were grown at 30°C and streaked on LB agar plates. When plates were incubated at 30°C, PMM77 and PMM78 strains grew as well as the WT. However, when plates were incubated at 42°C, a nonpermissive temperature for plasmid replication, both recombinant strains exhibited growth inhibition in contrast to the WT (Figure 2A). These findings firmly established that AcpS and PptT are required for the survival of *M. smegmatis* and therefore activate proteins involved in essential biosynthesis pathways. These results also demonstrated that the two PPTases can not substitute for each other.

### Example 4 : C. glutamicum ΔpptT::km and ΔacpS::km mutants display different microbiological phenotype.

Among the PPTase subtrastes, those responsible for the synthesis of fatty acids and mycolic acids are known to be essential for growth of *M. smegmatis* in laboratory conditions. We thus anticipated that both PPTases were involved in at least one of these metabolic pathways. Therefore, to study the role played by AcpS and PptT in fatty acids and mycolic acids biosynthesis, we switched to *C. glutamicum,* another bacterial model. Indeed, this bacterial species exhibits a cell envelope closely related to the one of mycobacteria , with a similar cell wall core (Daffé, 2005). However, we demonstrated in a previous work that this strain is more tolerant than the mycobacterial strain to mutation in genes involved in lipid metabolism (Portevin et al., 2004). To investigate the role of the two PPTases, we attempted to knockout the corresponding genes in C. *glutamicum.* Two alleles of *acpS* and *pptT,* disrupted by a Km resistant gene, were transferred on a non-replicative plasmid in C. *glutamicum* and transformants were selected on Km. For *pptT,* several Km resistant clones were screened by PCR and one clone that gave an amplification pattern consistent with the insertion of the Km cassette within *pptT* was retained for further analysis. Interestingly this *ΔpptT::km* mutant displayed phenotypic changes similar to those observed for the C. *glutamicum Δpks13* mutant which lacks an essential enzyme for the production of corynomycolates (Portevin et al., 2004). On BHI agar plates, the *ΔpptT::km* mutant exhibited small and rough colonies instead of large and shiny colonies for the WT. In addition this mutant grew much slower than the WT in liquid media and cells aggregated strongly whereas, under the same conditions WT cells grew well dispersed (data not shown). These phenotypic modifications were completely reverted when the mutant strain was transformed with plasmid pCGL-pptTcg which contains a functional copy of *pptT* from *C. glutamicum,* indicating that the observed phenotype of the mutant strain were due to the deletion of the *pptT* gene.

For *acpS,* the same strategy gave *ΔacpS::km* mutants, only when the growth medium was supplemented with fatty acids. A phenotypical analysis confirmed that these C. *glutamicum ΔacpS::km* mutants were unable to grow on solid or liquid BHI medium except when medium were supplemented with sodium oleate and Tween 40. Under these conditions, no phenotypical differences were observed between the WT and *ΔacpS::km* recombinant strain (data not shown). This auxotrophy for oleic acid suggested that disruption of *acpS* in C. *glutamicum* affects the biosynthesis of fatty acids. The transfer of pCGL-acpScg, a corynebacterial plasmid carrying a functional *acpS* gene from C. *glutamicum,* into the *ΔacpS::km* mutant strain restored the capacity for the bacteria cells to grow on solid or liquid medium without oleate indicating that the phenotypic changes observed between the mutated and the WT strains relied solely on the disruption of the *acpS* gene.

Altogether these experiments showed that disruption of *acpS* and *pptT* in C. *glutamicum* led to strains exhibiting different phenotypes. The oleic acid auxotrophy of the *ΔacpS::km* mutant suggested that *acpS* is involved in fatty acids biosynthesis. In contrast the *pptT* gene was not required for the viability of C. *glutamicum* but the morphological alterations of the *ΔpptT::km* mutant indicated cell envelope modifications consistent with absence of mycolate production.

### Example 5 : AcpS and PptT PPTases are respectively involved in fatty acids and mycolic acids biosynthesis in C. glutamicum.

To further characterize the phenotypes induced by the disruption of *acpS* and *pptT,* we carried out biochemical characterization of fatty acids and corynomycolates produced by the two C. *glutamicum* recombinant strains.

Cultures of *C. glutamicum* WT, *ΔacpS::km* mutant and *ΔacpS::km:*pCGL-acpScg complemented strain were grown to exponential phase (in presence of oleic acid and Tween 40 for the mutant strain) and fatty acids were released from bacteria by saponification of whole cells. Thin layer chromatography (TLC) analysis showed that the *ΔacpS::km* mutant synthesized corynomycolates at the same level than the WT or the complemented strain (Figure 3A). When fatty acid methyl ethers were analysed by GC, we found that the *ΔacpS::km* mutant strain exhibited large amounts of C₁₆ and C_{18:1} fatty acids, the precursors of corynomycolates. Since this mutant was auxotrophic for fatty acids, these lipids probably originated from oleic acid and Tween 40 added to the medium. In contrast to the WT (Figure 3B, upper left) and the complemented strains (data not shown) which synthetized various forms of corynomycolates, the *ΔacpS::km* mutant strain produced only one detectable species of corynomycolic acid (Figure 3B, upper right). A further characterization of this lipid by GC-MS analysis revealed that it corresponds a C_{36:2} corynomycolate (Figure 3C) indicating that the mutant was able to condense two C_{18:1} fatty acids provided by the exogenous addition of oleic acid into the medium. The absence of C₃₂ and C₃₄ corynomycolates in the cell envelope of the mutant is surprising since C₁₆ fatty acids is available in the growth medium. However, it was previously shown that when large amount of oleate are supplied to C. *glutamicum,* there is a change in mycolate composition toward C_{36:2} corynomycolate (Radmacher et al., 2005).

Unlike mycobacteria which possess two fatty acid synthase systems (Fas-I and Fas-II), C. *glutamicum* lacks the Fas-II system but has two Fas-I proteins (Fas-IA and Fas-IB). It was shown that Fas-IA is essential for growth but not Fas-IB (Radmacher et al., 2005). The fact that disruption of *acpS* in C. *glutamicum* affects the biosynthesis of fatty acids means that the Fas-IA enzyme is inactive in the *ΔacpS::km* mutant strongly suggesting that AcpS is responsible for the 4'-phosphopantetheinylation of this enzyme. These results also established that PptT cannot complement the lack of AcpS to perform this reaction in *C*. *glutamicum* providing a strong support to the model that the two PPTases are not redundant. Moreover the ability for the recombinant strain to sustain corynomycolates synthesis in presence of exogenous fatty acids implies that the AcpS PPTase is not required for the posttranslational modification of the condensing enzyme Pks13.

We next investigated whether phenotypic changes observed with the *ΔpptT::km* mutant reflect cell wall modifications. TLC and GC analyses revealed that disruption of *pptT* in *C. glutamicum* abolished the production of corynomycolates (Figure 3A and 3B, lower left). Complementation of the mutation by reintroduction of a WT allele in the mutant, fully restored the production of all classes of corynomycolates (C₃₂, C₃₄, C₃₆) confirming that the differences observed between the mutant and the WT strains were due to the disruption of the *pptT* gene (data not shown). We also found that the *ΔpptT::km* mutant produced large amounts of C₁₆ and C₁₈ fatty acids (Figure 3B). Thus, it appears that PptT is not required for the production of fatty acids in corynebacteria cells and is therefore not involved in the posttranslational modification of the Fas-IA enzyme. In addition the inability for the mutant strain to synthetize mycolic acids from fatty acids suggests that PptT is involved in the activation of the condensing enzyme Pks13 protein.

Taken together, these experiments established that the AcpS and PptT display different functions in *C*. *glutamicum,* the former being involved in the Fas-I activation and the latter in the modification of Pks13.

### Example 6 : Biochemical characterization of the 4'-phosphopantetheinylation of Pks13 and Fas-I in C. glutamicum.

The phenotypic and biochemical analysis of the two *C*. *glutamicum* mutants provide indirect evidences that AcpS and PptT activate Fas-I and Pks13 respectively. In order to directly demonstrate the specificity of the two PPTases, we designed an experiment to label the P-pant arm and to visualize its transfer onto the various protein substrates. The various *C*. *glutamicum* WT and recombinant strains were grown on CGXII minimal medium in presence of β-[β-¹⁴C]alanine, a precursor of CoA. The principle of this experiment was to generate a pool of CoA harbouring a ¹⁴C radiolabeled 4'-phosphopantetheine prosthetic group within the bacteria. This pool may then serve as substrate for the two PPTases to specifically label the 4'-phosphopantetheinylated proteins. After growth, cells were harvested, lyzed and proteins from cellular extracts were separated by SDS-PAGE before Coomassie blue staining and autoradiography to visualize the radiolabelled proteins.

When this experiment was performed with the WT *C. glutamicum,* two protein bands were labeled exhibiting apparent molecular weight of 170 kDa and higher than 220 kDa (Figure 4A, upper). The identity of the smaller protein was established by the use of a *Δpks13* mutant which no longer exhibits the labeled 170 kDa band. A search in the genomic database of *C. glutamicum* ATCC13032 for the presence of large proteins which must be converted by the covalent attachment of the P-pant group revealed only Fas-IA and Fas-IB (315 and 317 kD) as being larger than 220 kDa. We thus concluded that the higher labelled protein band corresponds to the Fas-I enzymes.

When the same experiments was performed with *ΔpptT* strain, Pks13 was no longer labelled (Figure 4A, upper). In contrast, the Fas-I enzymes were still activated in this strain. The absence of labelling was not due to expression deficiency since Coomassie blue staining of the polyacrylamide gel revealed that Pks13 was present in the cell extract from the *ΔpptT* mutant, although unlabelled (Figure 4A, lower). When the experiments were performed with the *ΔacpS* mutant, the opposite labelling pattern was observed: Pks13 was labelled but not the Fas-I enzymes.

Therefore, these experiments established for the first time the repertoire of protein substrate for the two PPTases of C. *glutamicum:* AcpS and PptT have a strict substrate specificity for the Fas-I enzymes and Pks13 respectively.

### Example 7 : AcpS and PptT display identical functions in mycobacteria and corynebacteria.

The above-described experiments established the function played by each PPTase in corynebacteria cells. To study if these proteins display similar functions in mycobacteria, we tested whether the disruption of either the *pptT* or the *acpS* gene in *M. smegmatis* could be complemented by the expression of the corresponding orthologs of C. *glutamicum.* To address this issue, two mycobacterial temperature-sensitive plasmids, named pC-acpScg and pC-pptTcg, harbouring the *acpS* and the *pptT* gene of *C. glutamicum,* respectively, were constructed. pC-acpScg was transferred in *M. smegmatis* PMM68 which is merodiploid for the *acpS* gene and pC-pptTcg in *M. smegmatis* PMM70, the merodiploid for the *pptT* gene. Several transformants were grown in LB at 30°C before plating on solid medium containing sucrose to induce the second recombination event between the two chromosomal alleles at either the *pptT* or the *acpS* loci. Several colonies, in which the WT chromosomal copy of *acpS* or *pptT* were replaced by the mutated allele, were selected for further analyses and named PMM84 (*ΔacpS::km*:pC-acpScg) and PMM85 (*ΔpptT::km*:pC-pptTcg).

PMM84 and PMM85 exhibited WT phenotype identical to the one obtained with mutants PMM77 (*ΔacpS::km*:pC-acpSms) and PMM78 (*ΔpptT::km*:pC-pptTms) which expressed AcpS and PptT of *M. smegmatis,* respectively. Both mutants strains sustained normal growth in LB liquid medium and on LB agar plates at 30°C but were unable to support growth on solid medium at 42°C, a non permissive temperature for plasmid replication (Figure 2B). These data demonstrated that the two PPTases of C. *glutamicum* are able to recognize and posttranslationally modify the same essential proteins in *M. smegmatis* than their mycobacterial orthologs indicating that AcpS and PptT have similar functions in both species.

A similar cross-complementation was observed when the PptT of *M. smegmatis* was expressed in the C. *glutamicum ΔpptT::km.* In contrast to *C. glutamicum ΔpptT::km* cells, the strain complemented with *pptT* from *M. smegmatis* showed no aggregation in liquid medium and grew as smooth shiny colonies on agar plates. In addition, analysis of fatty acids by TLC and GC after saponification of whole cells revealed that the production of corynomycolates had been restored at the same level than in the WT strain (Figure 3A and 3B, lower right). As stated above, the unusual phenotype of the C. *glutamicum ΔpptT::km* strain results from the lack of phosphopantetheinylation of Pks13. Thus it appears that the *M. smegmatis* PptT can activate the Pks13 of C. *glutamicum* suggesting that this protein performs the same catalytic reaction in *M. smegmatis.* Altogether these cross-complementation experiments show that PptT and AcpS PPTases have similar functions in the biosynthesis of fatty acids and mycolic acids of mycobacteria and corynebacteria,.

### Example 8 : PptT is responsible for the 4'-phosphopantetheinylation of type-I Pks in M. tuberculosis.

We demonstrated that AcpS is involved in the activation of Fas-I in corynebacteria and mycobacteria whereas PptT catalyzes the posttranslational modification of Pks13. This protein is the only type-I Pks encoded by the corynebacterial genome. However, the repertoire of type-I Pks is much larger in mycobacteria. These additional proteins are involved in the formation of extractable lipids, such as DIM or PGL-tb in *M. tuberculosis,* which are key virulence factors. We then wonder whether PptT is also involved in the activation of the other type-I Pks of *M. tuberculosis.*

To address this question, various *M. tuberculosis* Pks were coproduced in *E. coli* with PptT of *M. tuberculosis* and we looked at the activation of these proteins using radiolabeling with β-[β-¹⁴C]alanine. In primilary experiments, we observed that the *E. coli* PPTase EntD was responsible for partial activation of some *M. tuberculosis* Pks (our unpublished data). To overcome this problem, we constructed an *E. coli* BL21(DE3) *entD*-disrupted strain by allelic exchange and carried out the same experiments in the new strain. The experiment was first performed with Pks 13 because we already knew that it is a substrate of PptT (see results above). Results revealed that Pks13 was labelled in cells coproducing Pks13 and PptT from *M. tuberculosis* but not in cells producing Pks13 alone (Figure 4B, upper). To rule out the possibility of a nonspecific labeling of Pks13, we constructed plasmid pWM35γ, a derivative of pWM35 expressing a Pks13 protein mutant in which the two serine residues (Ser55 and Ser1266) responsible for the attachment of the P-pant moiety of CoA within the N- and C-terminal ACP domains of Pks13, have been substituted by alanine residues. When this mutant protein was coproduced with PptT in *E. coli* BL21 *ΔentD,* we did not observe any radiolabelling in our assay indicating that the signal obtained with Pks13 specifically resulted from the attachment of the P-pant moiety of CoA to the catalytic serines of Pks13 (Figure 4B). These experiments provided a direct evidence that Pks13 is a substrate of PptT in *M. tuberculosis.*

Once the test functional, we then looked at the activation of other type-I Pks by PptT. Five different type-I Pks were independently coproduced with PptT in *E. coli* BL21 *ΔentD* and in all cases, transfer of the radiolabeled P-pant was detected (Figure 4C, upper). The signals were weak but reproducibly detected. In contrast, none of these Pks was found to be labelled in *E. coli* strains lacking the mycobacterial PptT PPTase. This absence of detectable activation may not be attributed to low level of protein expression as indicated by the Coomassie Blue staining of the protein gel which demonstrated that type-I Pks were produced at high level without the PptT PPTase (Figure 4C, lower).

According to this, it can be concluded that PptT is not only responsible for the activation of Pks13 in *M. tuberculosis* but is also required for the modification of other type-I Pks involved in the biosynthesis of lipids required for virulence.

### Discussion

Given the crucial role of cell envelope lipids in the biology of *M. tuberculosis,* tremendous efforts have been made during the last decades to decipher the cellular processes leading to the production and translocation of these components. From these efforts, the concept has emerged that the unique lipid structures found in mycobacteria are synthesized by the combined action of Fas systems and type-I Pks: both classes of enzymes which have to be converted from inactive apo-forms to functional holo-forms. In this study, we have examined the role of two PPTases in the posttranslational modification of these biosynthetic enzymes. We provided direct evidence that the two PPTases activate each a defined subset of protein substrates in mycobacteria and corynebacteria and are both essential for the viability of mycobacteria. These results have important implications for our understanding of the lipids metabolism in mycobacteria and related bacteria. They demonstrate the central role played by the two PPTases in the biology of these microorganisms, defining new promising drug targets for fighting tuberculosis.

Concerning the lipid metabolism in mycobacteria, we propose a model in which AcpS is dedicated to the posttranslational modification of Fas-I and the AcpM subunit of Fas-II whereas PptT activates the numerous type-I Pks and NRPS of *M. tuberculosis* (Figure 5). This model is also true for *Corynebacteria spp.* but the number of proteins to modify is smaller than in mycobacteria, and especially *M. tuberculosis.* Several lines of evidence are consistent with this model. First, the results presented here clearly established that the two mycobacterial or corynebacterial PPTases are not redundant and exhibit different repertoire of substrates. Indeed, the two mycobacterial PPTases were independently essential for the viability of mycobacteria. C. *glutamicum* deleted for *acpS* exhibited fatty acid auxotrophy and no activation of Fas-I but remained proficient for mycolic acid formation and activation of Pks13. In contrast, deletion of *pptT* led to a C. *glutamicum* mutant unable to synthesize mycolic acid and to activate Pks13 while still able to produce C₁₆-C₁₈ fatty acid and to modify Fas-I. So, these data demonstrated that Fas-I and Pks13 are substrates of AcpS and PptT, respectively. Second, our results showed that five other type-I Pks of *M. tuberculosis,* different in size and in their domain contents, are also activated by PptT. Although we cannot formally rule out the possibility that these Pks are also substrates of AcpS, the lack of redundancy of the two PPTases argues against this hypothesis. Therefore, our findings strongly support the model that all the type-I Pks of *M. tuberculosis* are activated by PptT. Third, a previous report established that, *in vitro,* PptT is able to 4'-phosphopantetheinylate MbtB and MbtE, two NRPS acting in mycobactins assembly (Quadri et al., 1998b). The activation of the two additional proteins, MbtF a peptide synthase and MbtD a polyketide synthase, encoded by the mycobactin gene cluster remains speculative due to the lack of experimental data but it can be inferred from our results and those of Quadri et al. (1998b) that both proteins are also activated by PptT. Finally, it was shown that AcpM exhibits a three-dimensional structure very similar to Acp from *E. coli* which is a substrate of AcpS (Wong et al., 2002). This protein, AcpM, can be activated by AcpS of *E. coli both in vitro and in vivo* supporting the model that this protein is also the substrate of AcpS in mycobacteria (Schaeffer et al., 2001; Wong et al., 2002). Therefore all the results obtained in this study or previously published support our model. This model is also consistent with previous observations made in other microorganisms showing that Sfp-type PPTases are usually functionally associated with secondary metabolism (Mootz et al., 2001).

Our results have also important implications for our understanding of the biology of mycobacteria and especially *M. tuberculosis.* Indeed, *M. tuberculosis* contains more than 20 proteins which have to be 4'-phosphopantetheinylated. Ours findings provided a definition of the substrate repertoire of each PPTase and showed that both enzymes are required for the formation of essential components for the viability of mycobacteria. Hence, the enzyme, Fas-I, which is activated by AcpS, catalyses the synthesis of C₁₆-C₁₈ fatty acids which are incorporated in the various lipid constituents of the plasma membrane. Synthesis of short fatty acids by Fas-I is also one of the first steps of the long biosynthesis pathway leading to the formation of mycolates, which are key structural elements of the mycobacterial cell wall skeleton (Daffé and Draper, 1998). This pathway includes also two other proteins AcpM and Pks13 activated by AcpS and PptT, respectively: AcpM is a subunit of the Fas-II system synthesizing the long meromycolate chain and Pks13 is the condensase catalysing the last condensation step of mycolate formation. Therefore, the two mycobacterial PPTases are required for mycolates formation. As a consequence, they are both essential for viability of mycobacteria.

In addition, these two enzymes are also required for the production of important virulence factors. For instance, the enzymes MbtB and MbtD-F activated by PptT are involved in the assembly of mycobactin siderophores that are required for growth within human macrophages (de Voss et al., 2000). Along the same line, Fas-I and seven type-I Pks, activated by AcpS and PptT respectively, are involved in the formation of DIM and PGL-tb, two complex lipids produced by a very limited number of mycobacterial species and two important virulence factors of *M. tuberculosis.* Indeed, DIM-less mutants are affected in their capacity to multiply within the host and to cause diseases (Cox et al., 1999). Similarly, a clinical isolates of *M. tuberculosis* exhibiting a hypervirulence phenotype in various animal model was shown to be attenuated by mutation in Pks15/1, an enzyme required for PGL-tb formation (Reed et al., 2004).

Thus the two PPTases appear to have central roles for the biology of the pathogen *M. tuberculosis* being required both for viability and pathogenesis. Other mycobacterial pathogens such as *M. leprae, M. ulcerans* or *M. avium* also possess orthologs of AcpS and PptT and all produce mycolates and lipid virulence factors. Therefore, the two mycobacterial PPTases are very promising targets for the development of drugs for fighting mycobacterial infections.

### Example 9 : PptT enzymatic assay

The PPTase activity consists in the transfer of the 4'-phosphopantetheine group (P-pant) from coenzyme A onto the acyl carrier protein (ACP) domain of Pks. The PptT activity is assayed by using a radioactive assay method as described previously for various PPTases including Sfp from *Bacillus subtilis* or AcpS from *E. coli* (Lambalot, Gehring et al. 1996; Quadri, Weinreb et al. 1998a; Mootz, Finking et al. 2001) and is adapted to the mycobacterial PptT PPTase. This method measures the incorporation of the ³H-labeled 4' phosphopantetheine group from (³H)coenzyme A into apoenzymes.

Typically, reaction mixtures containing MgCl₂, the PptT protein, the protein substrate (apoenzyme) and the (³H)CoenzymeA cosubstrate is incubated for 30 min at 37°C. Reactions are quenched by addition of 10% trichloroacetic acid (TCA) and BSA added as a carrier. Precipitated proteins are collected by centrifugation and the resulting pellets are washed with TCA and dissolved in 1M Tris base. The redissolved proteins are mixed with liquid scintillation cocktail and the amount of radioactivity incorporated into the protein substrate quantified using a liquid scintillation analyzer.

The PptT protein used in these assays is fused to the MBP (Maltose Binding Protein) protein at its N-terminal extremity. This MBP-PptT fusion protein is overexpressed in *E. coli* strain BL21 and partially purified by affinity chromatography using an amylose resin.

Different protein substrates (apoenzymes) consisting of either whole mycobacterial Pks proteins or ACP domains of these Pks are tested in order to select the most suitable substrate for the test. As examples of Pks proteins that can be used in this test, Pks 13 and Mas (*mycocerosic acid synthase,* which is implicated in the synthesis of mycocerosic acids in mycobacteria producing DIMs and PGLs) can be cited. They can be produced fused to a carboxy-terminal His tag in a engineered *E. coli* strain deleted for the *entD* gene which encodes an *E. coli* PPTase to avoid background activation (production of apo forms). Such His tagged-proteins are purified by affinity chromatography (Nickel column) followed by size exclusion chromatography.

Once the test is functional, the (³H)CoenzymeA is substituted by CoenzymeA analogs harboring either a fluorescent or a biotinylated phosphopantetheine group to design an enzymatic assay suitable for high throughput screening of libraries of compounds. Several studies have demonstrated that these modified substrates may be efficiently transferred on various protein substrates by Sfp (La Clair, Foley et al. 2004; Yin, Liu et al. 2004).

### REFERENCES

Asselineau, C., Asselineau, J., Laneelle, G., and Laneelle, M.A. (2002). The biosynthesis of mycolic acids by Mycobacteria: current and alternative hypotheses. Prog. Lipid Res. 41, 501-523.
Banerjee, A., Dubnau, E., Quemard, A., Balasubramanian, V., Um, K.S., Wilson, T., Collins, D., de Lisle, G., and Jacobs, W.R., Jr. (1994). inhA, a gene encoding a target for isoniazid and ethionamide in Mycobacterium tuberculosis. Science 263, 227-230.
Camacho, L.R., Ensergueix, D., Perez, E., Gicquel, B., and Guilhot, C. (1999). Identification of a virulence gene cluster of Mycobacterium tuberculosis by signature-tagged transposon mutagenesis. Mol. Microbiol. 34, 257-267.
Cane, D.E., and Walsh, C.T. (1999). The parallel and convergent universes of polyketide synthases and nonribosomal peptide synthetases. Chem. Biol. 6, R319-325.
Cole, S. T., Brosch, R., Parkhill, J., Garnier, T., Churcher, C., Harris, D., Gordon, S. V., Eiglmeier, K., Gas, S., Barry, C. E., 3rd, et al. (1998). Deciphering the biology of Mycobacterium tuberculosis from the complete genome sequence. Nature 393, 537-544.
Constant, P., Perez, E., Malaga, W., Laneelle, M.A., Saurel, O., Daffé, M., and Guilhot, C. (2002). Role of the pks15/1 gene in the biosynthesis of phenolglycolipids in the Mycobacterium tuberculosis complex. Evidence that all strains synthesize glycosylated p-hydroxybenzoic methly esters and that strains devoid of phenolglycolipids harbor a frameshift mutation in the pks15/1 gene. J. Biol. Chem. 277, 38148-38158.
Cox, J.S., Chen, B., McNeil, M., and Jacobs, W.R., Jr. (1999). Complex lipid determines tissue-specific replication of Mycobacterium tuberculosis in mice. Nature 402, 79-83.
Daffé, M., and Draper, P. (1998). The envelope layers of mycobacteria with reference to their pathogenicity. Adv. Microb. Physiol. 39, 131-203.
Daffé, M. (2005). The cell envelope of Corynebacteria. In Handbook of Corynebacterium glutamicum, L. Eggeling and M. Bott, ed. (Taylor and Francis, CRC Press), pp. 121-148.
De Voss, J.J., Rutter, K., Schroeder, B.G., and Barry, C.E., 3rd (1999). Iron acquisition and metabolism by mycobacteria. J. Bacteriol. 181, 4443-4451.
De Voss, J.J., Rutter, K., Schroeder, B.G., Su, H., Zhu, Y., and Barry, C.E., 3rd (2000). The salicylate-derived mycobactin siderophores of Mycobacterium tuberculosis are essential for growth in macrophages. Proc. Natl. Acad. Sci. USA 97, 1252-1257.
Dubey, V.S., Sirakova, T.D., and Kolattukudy, P.E. (2002). Disruption of msl3 abolishes the synthesis of mycolipanoic and mycolipenic acids required for polyacyltrehalose synthesis in Mycobacterium tuberculosis H37Rv and causes cell aggregation. Mol. Microbiol. 45, 1451-1459.
Fichtlscherer, F., Wellein, C., Mittag, M., and Schweizer, E. (2000). A novel function of yeast fatty acid synthase. Subunit alpha is capable of self-pantetheinylation. Eur. J. Biochem. 267, 2666-2671.
Flugel, R.S., Hwangbo, Y., Lambalot, R.H., Cronan, J.E., Jr., and Walsh, C.T. (2000). Holo-(acyl carrier protein) synthase and phosphopantetheinyl transfer in Escherichia coli. J. Biol. Chem. 275, 959-968.
Guilhot, C., Otal, I., Van Rompaey, I., Martin, C., and Gicquel, B. (1994). Efficient transposition in mycobacteria: construction of Mycobacterium smegmatis insertional mutant libraries. J. Bacteriol. 176, 535-539.
Keating, T.A., and Walsh, C.T. (1999). Initiation, elongation, and termination strategies in polyketide and polypeptide antibiotic biosynthesis. Curr. Opin. Chem. Biol. 3, 598-606.
Keilhauer, C., Eggeling, L., and Sahm, H. (1993). Isoleucine synthesis in Corynebacterium glutamicum: molecular analysis of the ilvB-ilvN-ilvC operon. J. Bacteriol. 175, 5595-5603.
La Clair, J.J., Foley, T.L., Schegg, T.R., Regan, C.M., and Burkart, M. D. (2004). Manipulation of carrier proteins in antibiotic biosynthesis. Chem Biol 11, 195-201.
Lambalot, R.H., Gehring, A.M., Flugel, R.S., Zuber, P., LaCelle, M., Marahiel, M.A., Reid, R., Khosla, C., and Walsh, C.T. (1996). A new enzyme superfamily - the phosphopantetheinyl transferases. Chem. Biol. 3, 923-936.
Laval, F., Laneelle, M.A., Deon, C., Monsarrat, B., and Daffé, M. (2001). Accurate molecular mass determination of mycolic acids by MALDI-TOF mass spectrometry. Anal. Chem. 73, 4537-4544.
Le Dantec, C., Winter, N., Gicquel, B., Vincent, V., and Picardeau, M. (2001). Genomic sequence and transcriptional analysis of a 23-kilobase mycobacterial linear plasmid: evidence for horizontal transfer and identification of plasmid maintenance systems. J. Bacteriol. 183, 2157-2164.
Minnikin, D.E., Kremer, L., Dover, L.G., and Besra, G.S. (2002). The methyl-branched fortifications of Mycobacterium tuberculosis. Chem. Biol. 9, 545-553.
Mofid, M.R., Finking, R., Essen, L.O., and Marahiel, M.A. (2004). Structure-based mutational analysis of the 4'-phosphopantetheinyl transferases Sfp from Bacillus subtilis: carrier protein recognition and reaction mechanism. Biochemistry 43, 4128-4136.
Mootz, H.D., Finking, R., and Marahiel, M.A. (2001). 4'-phosphopantetheine transfer in primary and secondary metabolism of Bacillus subtilis. J. Biol. Chem. 276, 37289-37298.
Onwueme, K.C., Vos, C.J., Zurita, J., Ferreras, J.A., Quadri, L.E.N. (2005). The dimycocerosate ester polyketide virulence factors of mycobacteria. Prog. Lipid Res., 44, 259-302.
Parris, K.D., Lin, L., Tam, A., Mathew, R., Hixon, J., Stahl, M., Fritz, C.C., Seehra, J., and Somers, W.S. (2000). Crystal structures of substrate binding to Bacillus subtilis holo-(acyl carrier protein) synthase reveal a novel trimeric arrangement of molecules resulting in three active sites. Structure Fold. Des. 8, 883-895.
Peyret, J.L., Bayan, N., Joliff, G., Gulik-Krzywicki, T., Mathieu, L., Schechter, E., and Leblon, G. (1993). Characterization of the cspB gene encoding PS2, an ordered surface-layer protein in Corynebacterium glutamicum. Mol. Microbiol. 9, 97-109.
Portevin, D., De Sousa-D'Auria, C., Houssin, C., Grimaldi, C., Chami, M., Daffé, M., and Guilhot, C. (2004). A polyketide synthase catalyzes the last condensation step of mycolic acid biosynthesis in mycobacteria and related organisms. Proc. Natl. Acad. Sci. USA 101, 314-319.
Quadri, L.E., Weinreb, P.H., Lei, M., Nakano, M.M., Zuber, P., and Walsh, C.T. (1998a). Characterization of Sfp, a Bacillus subtilis phosphopantetheinyl transferase for peptidyl carrier protein domains in peptide synthetases. Biochemistry 37, 1585-1595.
Quadri, L.E., Sello, J., Keating, T.A., Weinreb, P.H., and Walsh, C.T. (1998b). Identification of a Mycobacterium tuberculosis gene cluster encoding the biosynthetic enzymes for assembly of the virulence-conferring siderophore mycobactin. Chem. Biol. 5, 631-645.
Quandt, J., and Hynes, M.F. (1993). Versatile suicide vectors which allow direct selection for gene replacement in gram-negative bacteria. Gene 127, 15-21.
Radmacher, E., Alderwick, L.J., Besra, G.S., Brown, A.K., Gibson, K.J., Sahm, H., and Eggeling, L. (2005). Two functional FAS-I type fatty acid synthases in Corynebacterium glutamicum. Microbiology 151, 2421-2427.
Reed, M.B., Domenech, P., Manca, C., Su, H., Barczak, A.K., Kreiswirth, B.N., Kaplan, G., and Barry, C.E., 3rd (2004). A glycolipid of hypervirulent tuberculosis strains that inhibits the innate immune response. Nature 431, 84-87.
Sambrook, J. and Russel, D.W. (2001). Molecular cloning: a laboratory manuel. (New York : Cold Spring Harbor Laboratory Press).
Schaeffer, M.L., Agnihotri, G., Kallender, H., Brennan, P.J., and Lonsdale, J.T. (2001). Expression, purification, and characterization of the Mycobacterium tuberculosis acyl carrier protein, AcpM. Biochim. Biophys. Acta 1532, 67-78.
Sirakova, T.D., Thirumala, A.K., Dubey, V.S., Sprecher, H., and Kolattukudy, P.E. (2001). The Mycobacterium tuberculosis pks2 gene encodes the synthase for the hepta- and octamethyl-branched fatty acids required for sulfolipid synthesis. J. Biol. Chem. 276, 16833-16839.
Snapper, S.B., Melton, R.E., Mustafa, S., Kieser, T., and Jacobs, W.R., Jr (1990). Isolation and characterization of efficient plasmid transformation mutants of Mycobacterium smegmatis. Mol. Microbiol. 4, 1911-1919.
Takayama, K., Wang, C., and Besra, G.S. (2005). Pathway to synthesis and processing of mycolic acids in Mycobacterium tuberculosis. Clin. Microbiol. Rev. 18, 81-101.
Walsh, C.T., Gehring, A.M., Weinreb, P.H., Quadri, L.E., and Flugel, R.S. (1997). Post-translational modification of polyketide and nonribosomal peptide synthases. Curr. Opin. Chem. Biol. 1, 309-315.
Weissman, K.J., Hong, H., Oliynyk, M., Siskos, A.P., and Leadlay, P.F. (2004). Identification of a phosphopantetheinyl transferase for erythromycin biosynthesis in Saccharopolyspora erythraea. Chembiochem. 5, 116-125.
Wong, H.C., Liu, G., Zhang, Y.M., Rock, C.O., and Zheng, J. (2002). The solution structure of acyl carrier protein from Mycobacterium tuberculosis. J. Biol. Chem. 277, 15874-15880.
Yin, J., Liu, F., Li, X., and Walsh, C.T. (2004). Labeling proteins with small molecules by site-specific posttranslational modification. J Am Chem Soc 126, 7754-7755.

## Claims

1. Use of a 4'phosphopantetheinyl transferase PptT protein from a pathogenic bacterium containing mycolic acids, as a target for *in vitro* screening compounds for identifying those inhibiting the biosynthesis of mycolic acids, wherein a compound is identified as inhibiting the biosynthesis of mycolic acids if it inhibits said PptT activity.

2. The use according to claim 1, wherein said PptT protein is from a bacterium selected in the group consisting of corynebacteria, mycobacteria, and nocardia.

3. The use according to claim 2, wherein said PptT protein is from a bacterium selected in the group consisting of *Corynebacterium diphtheriae, Corynebacterium minutissimum, Corynebacterium pseudotuberculosis, Mycobacterium africanum, Mycobacterium avium, Mycobacterium chelonae, Mycobacterium fortuitum, Mycobacterium kansasii, Mycobacterium leprae, Mycobacterium malmoense, Mycobacterium marinum, Mycobacterium microti, Mycobacterium paratuberculosis, Mycobacterium scrofulaceum*, *Mycobacterium simiae, Mycobacterium szulgai, Mycobacterium tuberculosis, Mycobacterium ulcerans, Mycobacterium xenopi, Nocardia asteroids, Nocardia brasiliensis, Nocardia farcinica, Nocardia nova,* and *Nocardia otitidiscaviarum.*

4. The use according to any of claims 1 to 3, wherein the amino acids sequence of said PptT protein is selected from the group consisting of SEQ Nos: 2 to 9.

5. An *in vitro* screening process for identifying compounds inhibiting the biosynthesis of mycolic acids, **characterized in that** the activity of a 4'phosphopantetheinyl transferase PptT protein from a pathogenic bacterium containing mycolic acids is measured in the presence or absence of said compounds, wherein a compound is identified as inhibiting the biosynthesis of mycolic acids if it inhibits said PptT activity.

6. The process according to claim 5, wherein said PptT activity is measured by (i) incubating an isolated PptT with a Pks protein or subunit thereof comprising an acyl carrier protein domain, and with a labelled Acetyl-CoA, (ii) precipitating said Pks protein or subunit thereof, and (iii) measuring the level of labelling of said precipitate.

7. The process according to claim 5 or claim 6, wherein said PptT is as defined in any of claims 1 to 4.

## Patentansprüche

1. Verwendung eines 4'-Phosphopantetheinyl-Transferase-PptT-Proteins aus einem pathogenen Bakterium, das Mykolsäuren enthält, als Target zum *In-vitro-*Screening von Verbindungen zum Identifizieren solcher, die die Biosynthese von Mykolsäuren inhibieren, wobei eine Verbindung als die Biosynthese von Mykolsäuren inhibierend identifiziert wird, wenn sie die PptT-Aktivität inhibiert.

2. Verwendung gemäß Anspruch 1, wobei das PptT-Protein aus einem Bakterium ist, das aus der Gruppe, bestehend aus Corynebakterien, Mykobakterien und Nocardien, ausgewählt ist.

3. Verwendung gemäß Anspruch 2, wobei das PptT-Protein aus einem Bakterium ist, das aus der Gruppe, bestehend aus *Corynebacterium diphtheriae, Corynebacterium minutissimum, Corynebacterium pseudotuberculosis, Mycobacterium africanum, Mycobacterium avium, Mycobacterium chelonae, Mycobacterium fortuitum, Mycobacterium kansasii, Mycobacterium leprae, Mycobacterium malmoense, Mycobacterium marinum, Mycobacterium microti, Mycobacterium paratuberculosis, Mycobacterium scrofulaceum, Mycobacterium simiae, Mycrobacterium szulgai, Mycobacterium tuberculosis, Mycobacterium ulcerans, Mycobacterium xenopi, Nocardia asteroids, Nocardia brasiliensis, Nocardia farcinica, Nocardia nova* und *Nocardia otitidiscaviarum* ausgewählt ist.

4. Verwendung gemäß einem der Ansprüche 1 bis 3, wobei die Aminosäuresequenz des PptT-Proteins aus der Gruppe, bestehend aus SEQ ID NOS:2 bis 9, ausgewählt ist.

5. *In-vitro*-Screening-Verfahren zur Identifizierung von Verbindungen, die die Biosynthese von Mykolsäuren inhibieren, **dadurch gekennzeichnet, dass** die Aktivität eines 4'-Phosphopantetheinyl-Transferase-PptT-Proteins aus einem pathogenen Bakterium, das Mykolsäuren enthält, in Gegenwart oder Abwesenheit der Verbindungen gemessen wird, wobei eine Verbindung als die Biosynthese von Mykolsäuren inhibierend identifiziert wird, wenn sie die PptT-Aktivität inhibiert.

6. Verfahren gemäß Anspruch 5, wobei die PptT-Aktivität gemessen wird, indem (i) ein isoliertes PptT mit einem Pks-Protein oder einer Untereinheit davon, umfassend eine Acylträger-Proteindomäne, und mit einem markierten Acetyl-CoA inkubiert wird, (ii) das Pks-Protein oder die Untereinheit davon präzipitiert wird und (iii) der Markierungslevel des Präzipitats gemessen wird.

7. Verfahren gemäß Anspruch 5 oder Anspruch 6, wobei das PptT wie in einem der Ansprüche 1 bis 4 definiert ist.

## Revendications

1. Utilisation d'une protéine PptT 4'-phosphopantéthéinyl transférase provenant d'une bactérie pathogène contenant des acides mycoliques comme cible pour un criblage *in vitro* de composés pour identifier ceux inhibant la biosynthèse des acides mycoliques, dans laquelle un composé est identifié comme inhibant la biosynthèse des acides mycoliques s'il inhibe l'activité de ladite PptT.

2. Utilisation selon la revendication 1, dans laquelle ladite protéine PptT provient d'une bactérie choisie dans le groupe constitué de *Corynebacterium,* de *Mycobacterium* et de *Nocardia.*

3. Utilisation selon la revendication 2, dans laquelle ladite protéine PptT provient d'une bactérie choisie dans le groupe constitué de *Corynebacterium diphtheriae, Corynebacterium minutissimum*, *Corynebacterium pseudotuberculosis, Mycobacterium africanum, Mycobacterium avium, Mycobacterium chelonae, Mycobacterium fortuitum, Mycobacterium kansasii, Mycobacterium leprae, Mycobacterium malmoense, Mycobacterium marinum, Mycobacterium microti, Mycobacterium paratuberculosis, Mycobacterium scrofulaceum, Mycobacterium simiae, Mycobacterium szulgai, Mycobacterium tuberculosis, Mycobacterium ulcerans, Mycobacterium xenopi, Nocardia asteroids, Nocardia brasiliensis, Nocardia farcinica, Nocardia nova* et *Nocardia otitidiscaviarum.*

4. Utilisation selon l'une quelconque des revendications 1 à 3, dans laquelle la séquence d'acides aminés de ladite protéine PptT est choisie dans le groupe constitué des SEQ ID N° : 2 à 9.

5. Procédé de criblage *in vitro* pour identifier des composés inhibant la biosynthèse des acides mycoliques, **caractérisé en ce que** l'activité d'une protéine PptT 4'-phosphopantéthéinyl transférase provenant d'une bactérie pathogène contenant des acides mycoliques est mesurée en présence ou en l'absence desdits composés, dans lequel un composé est identifié comme inhibant la biosynthèse des acides mycoliques s'il inhibe l'activité de ladite PptT.

6. Procédé selon la revendication 5, dans lequel l'activité de ladite PptT est mesurée par (i) incubation d'une PptT isolée avec une protéine Pks ou une sous-unité de celle-ci comprenant un domaine de protéine porteur d'acyle et avec une Acétyl-CoA marquée, (ii) précipitation de ladite protéine Pks ou de la sous-unité de celle-ci, et (iii) mesure du niveau de marquage dudit précipité.

7. Procédé selon la revendication 5 ou la revendication 6, dans lequel ladite PptT est telle que définie dans l'une quelconque des revendications 1 à 4.


### SEQUENCE LISTING

<110> CENTRE NATIONAL DE LA RECHERCHE SCIENTIFIQUE
<120> Use of a 4'-Phosphopantetheinyl Transferase as a target for
   identifying antibiotic molecules.
   CHALUT, Christian
   GUILHOT, Christophe
<130> BLO/VMA/ahF644/132EP
<160> 9
<170> Patentln version 3.3
<210> 1
   <211> 684
   <212> DNA
   <213> Microbacterium sp.
<220>
   <221> misc_feature
   <223> Coding sequence of the PptT from M. tuberculosis strain H37Rv,
   Rv2794c
<400> 1
   atgacggtag gcacgctggt ggcgtcggtg ttgccggcga ccgtgttcga ggatttggcg 60
   tatgccgagt tgtactccga cccacccggt ctcaccccgc tgcccgagga ggcgccgttg 120
   atcgcacgat cggttgccaa gcggcgcaac gaattcatca ccgtgcgtca ctgcgcccgc 180
   atcgcgctgg accagctcgg tgtgccgccg gcgccgatcc tcaagggcga caagggcgaa 240
   ccgtgctggc ccgacggcat ggtcggtagc ctcacccact gcgccggtta ccgcggcgcg 300
   gttgtcggac gcagggatgc ggtgcgttcc gtgggcatcg acgccgaacc gcacgacgtg 360
   ttgcccaatg gtgtgctgga tgcgatcagc ctgccggccg agcgcgccga catgccccgc 420
   accatgccag cggcgttgca ttgggatcga atcctgttct gcgccaagga agcaacgtac 480
   aaggcgtggt ttccgctgac caagaggtgg ctgggtttcg aggacgcgca catcacgttc 540
   gaaaccgata gcaccggctg gacgggtcgc ttcgtctccc gtatcctcat cgacgggtcc 600
   accctgtcgg gtccgccgct gacaacgctg cggggacgct ggtcggttga gcgcggactg 660
   gtgctgaccg cgatcgtgct atga 684
<210> 2
   <211> 227
   <212> PRT
   <213> Microbacterium sp.
<220>
   <221> misc_feature
   <223> PptT from M. tuberculosis strain H37Rv
<400> 2
   Met Thr Val Gly Thr Leu Val Ala Ser Val Leu Pro Ala Thr Val Phe
   1 5 10 15
   Glu Asp Leu Ala Tyr Ala Glu Leu Tyr Ser Asp Pro Pro Gly Leu Thr
   20 25 30
   Pro Leu Pro Glu Glu Ala Pro Leu Ile Ala Arg Ser Val Ala Lys Arg
   35 40 45
   Arg Asn Glu Phe Ile Thr Val Arg His Cys Ala Arg Ile Ala Leu Asp
   50 55 60
   Gln Leu Gly Val Pro Pro Ala Pro Ile Leu Lys Gly Asp Lys Gly Glu
   65 70 75 80
   Pro Cys Trp Pro Asp Gly Met Val Gly Ser Leu Thr His Cys Ala Gly
   85 90 95
   Tyr Arg Gly Ala Val Val Gly Arg Arg Asp Ala Val Arg Ser Val Gly
   100 105 110
   Ile Asp Ala Glu Pro His Asp Val Leu Pro Asn Gly Val Leu Asp Ala
   115 120 125
   Ile Ser Leu Pro Ala Glu Arg Ala Asp Met Pro Arg Thr Met Pro Ala
   130 135 140
   Ala Leu His Trp Asp Arg Ile Leu Phe Cys Ala Lys Glu Ala Thr Tyr
   145 150 155 160
   Lys Ala Trp Phe Pro Leu Thr Lys Arg Trp Leu Gly Phe Glu Asp Ala
   165 170 175
   His Ile Thr Phe Glu Thr Asp Ser Thr Gly Trp Thr Gly Arg Phe Val
   180 185 190
   Ser Arg Ile Leu Ile Asp Gly Ser Thr Leu Ser Gly Pro Pro Leu Thr
   195 200 205
   Thr Leu Arg Gly Arg Trp Ser Val Glu Arg Gly Leu Val Leu Thr Ala
   210 215 220
   Ile Val Leu
   225
<210> 3
   <211> 227
   <212> PRT
   <213> Microbacterium sp.
<220>
   <221> misc_feature
   <223> PptT from Mycobacterium tuberculosis strain CDC1551
<400> 3
225
<210> 4
   <211> 227
   <212> PRT
   <213> Microbacterium sp.
<220>
   <221> misc_feature
   <223> PptT from Mycobacterium Bovis
<400> 4
   Met Thr Val Gly Thr Leu Val Ala Ser Val Leu Pro Ala Thr Val Phe
   1 5 10 15
   Glu Asp Leu Ala Tyr Ala Glu Leu Tyr Ser Asp Pro Pro Gly Leu Thr
   20 25 30
   Pro Leu Pro Glu Glu Ala Pro Leu Ile Ala Arg Ser Val Ala Lys Arg
   35 40 45
   Arg Asn Glu Phe Ile Thr Val Arg His Cys Ala Arg Ile Ala Leu Asp
   50 55 60
   Gln Leu Gly Val Pro Pro Ala Pro Ile Leu Lys Gly Asp Lys Gly Glu
   65 70 75 80
   Pro Cys Trp Pro Asp Gly Val Val Gly Ser Leu Thr His Cys Ala Gly
   85 90 95
   Tyr Arg Gly Ala Val Val Gly Arg Arg Asp Ala Val Arg Ser Val Gly
   100 105 110
   Ile Asp Ala Glu Pro His Asp Val Leu Pro Asn Gly Val Leu Asp Ala
   115 120 125
   Ile Ser Leu Pro Ala Glu Arg Ala Asp Met Pro Arg Thr Met Pro Ala
   130 135 140
   Ala Leu His Trp Asp Arg Ile Leu Phe Cys Ala Lys Glu Ala Thr Tyr
   145 150 155 160
   Lys Ala Trp Phe Pro Leu Thr Lys Arg Trp Leu Gly Phe Glu Asp Ala
   165 170 175
   His Ile Thr Phe Glu Thr Asp Ser Thr Gly Trp Thr Gly Arg Phe Val
   180 185 190
   Ser Arg Ile Leu Ile Asp Gly Ser Thr Leu Ser Gly Pro Pro Leu Thr
   195 200 205
   Thr Leu Arg Gly Arg Trp Ser Val Glu Arg Gly Leu Val Leu Thr Ala
   210 215 220
   Ile Val Leu
   225
<210> 5
   <211> 227
   <212> PRT
   <213> Microbacterium sp.
<220>
   <221> misc_feature
   <223> PptT from Mycobacterium leprae
<400> 5
   Met Thr Val Ser Met Leu Val Ser Ser Val Leu Pro Asp Tyr Ala Ser
   1 5 10 15
   Gln Asp Leu Glu Tyr Ala Glu Leu Tyr Ser Asp Pro Pro Gly Leu Thr
   20 25 30
   Pro Leu Pro Glu Glu Glu Leu Leu Ile Ala Lys Ser Val Ala Lys Arg
   35 40 45
   Arg Asn Glu Phe Ile Thr Ala Arg Tyr Cys Ala Arg Ile Ala Leu Gly
   50 55 60
   Arg Leu Arg Val Pro Pro Val Pro Ile Leu Lys Gly Asp Lys Gly Glu
   65 70 75 80
   Pro Cys Trp Pro Asp Gly Val Val Gly Ser Leu Thr His Cys Ser Gly
   85 90 95
   Tyr Arg Gly Ala Val Val Gly Arg Ser Ala Ala Val Arg Ser Val Gly
   100 105 110
   Ile Asp Ala Glu Pro His Glu Met Leu Pro Asn Gly Val Leu Asp Val
   115 120 125
   Ile Ser Leu Pro Glu Glu Arg Ser Glu Met Arg Arg Lys Leu Pro Ser
   130 135 140
   Val Leu Tyr Trp Asp Arg Ile Leu Phe Cys Ala Lys Glu Ala Thr Tyr
   145 150 155 160
   Lys Ala Trp Phe Pro Leu Thr Lys Arg Trp Leu Gly Phe Glu Asp Ala
   165 170 175
   His Ile Thr Phe Asp Val Asp Asn Leu Gly Ser Ser Gly Gly Phe Val
   180 185 190
   Ser Arg Ile Leu Val Asp Gly Ser Ala Leu Ser Gly Pro Pro Leu Thr
   195 200 205
   Val Leu Thr Gly Arg Trp Ser Val Asp Arg Gly Leu Val Leu Thr Ala
   210 215 220
   Ile Val Leu
   225
<210> 6
   <211> 227
   <212> PRT
   <213> Microbacterium sp.
<220>
   <221> misc_feature
   <223> PptT from Mycobacterium marinum
<400> 6
   Val Thr Ile Ser Met Leu Val Ser Ser Val Leu Pro Gly Thr Val Val
   1 5 10 15
   Asp Asp Leu Ala Tyr Ala Glu Leu Tyr Ser Asp Pro Pro Gly Leu Val
   20 25 30
   Pro Leu Pro Glu Glu Glu Pro Leu Ile Ala Arg Ser Val Ala Lys Arg
   35 40 45
   Arg Asn Glu Phe Ile Thr Val Arg His Cys Ala Arg Val Ala Leu Gly
   50 55 60
   Asp Leu Gly Val Pro Pro Val Pro Ile Leu Lys Gly Asp Lys Gly Gln
   65 70 75 80
   Pro Cys Trp Pro Asp Gly Val Val Gly Ser Leu Thr His Cys Ser Gly
   85 90 95
   Tyr Arg Gly Ala Val Val Gly Arg Ser Ala Ala Val Arg Ser Val Gly
   100 105 110
   Ile Asp Ala Glu Pro His Asp Val Leu Pro Asn Gly Val Leu Asp Ala
   115 120 125
   Ile Ser Leu Pro Glu Glu Arg Asp Glu Ile Pro Ser Ala Met Pro Asp
   130 135 140
   Gly Leu His Trp Asp Arg Ile Leu Phe Cys Ala Lys Glu Ala Thr Tyr
   145 150 155 160
   Lys Val Trp Phe Pro Leu Thr Asn Arg Trp Leu Gly Phe Glu Asp Ala
   165 170 175
   His Ile Thr Phe Glu Ala Asp Asp Ser Gly Arg Thr Gly Arg Phe Val
   180 185 190
   Ser Arg Ile Leu Ile Asp Pro Ser Ala Leu Trp Gly Pro Pro Leu Thr
   195 200 205
   Thr Leu His Gly Arg Trp Ser Val Glu Arg Gly Leu Val Leu Thr Ala
   210 215 220
   Ile Val Leu
   225
<210> 7
   <211> 227
   <212> PRT
   <213> Microbacterium sp.
<220>
   <221> misc_feature
   <223> PptT from Mycobacterium microti
<400> 7
   Met Thr Val Gly Thr Leu Val Ala Ser Val Leu Pro Ala Thr Val Phe
   1 5 10 15
   Glu Asp Leu Ala Tyr Ala Glu Leu Tyr Ser Asp Pro Pro Gly Leu Thr
   20 25 30
   Pro Leu Pro Glu Glu Ala Pro Leu Ile Ala Arg Ser Val Ala Lys Arg
   35 40 45
   Arg Asn Glu Phe Ile Thr Val Arg His Cys Ala Arg Ile Ala Leu Asp
   50 55 60
   Gln Leu Gly Val Pro Pro Ala Pro Ile Leu Lys Gly Asp Lys Gly Glu
   65 70 75 80
   Pro Cys Trp Pro Asp Gly Val Val Gly Ser Leu Thr His Cys Ala Gly
   85 90 95
   Tyr Arg Gly Ala Val Val Gly Arg Arg Asp Ala Val Arg Ser Val Gly
   100 105 110
   Ile Asp Ala Glu Pro His Asp Val Leu Pro Asn Gly Val Leu Asp Ala
   115 120 125
   Ile Ser Leu Pro Ala Glu Arg Ala Asp Met Pro Arg Thr Met Pro Ala
   130 135 140
   Ala Leu His Trp Asp Arg Ile Leu Phe Cys Ala Lys Glu Ala Thr Tyr
   145 150 155 160
   Lys Ala Trp Phe Pro Leu Thr Lys Arg Trp Leu Gly Phe Glu Asp Ala
   165 170 175
   His Ile Thr Phe Glu Thr Asp Ser Thr Gly Trp Thr Gly Arg Phe Val
   180 185 190
   Ser Arg Ile Leu Ile Asp Gly Ser Thr Leu Ser Gly Pro Pro Leu Thr
   195 200 205
   Thr Leu Arg Gly Arg Trp Ser Val Glu Arg Gly Leu Val Leu Thr Ala
   210 215 220
   Ile Val Leu
   225
<210> 8
   <211> 225
   <212> PRT
   <213> Microbacterium sp.
<220>
   <221> misc_feature
   <223> PptT from Mycobacterium avium subsp paratuberculosis
<400> 8
   Met Thr Gly Thr Leu Val Ser Ser Val Leu Pro Ala Ser Asp Ala Leu
   1 5 10 15
   Ala Tyr Ser Glu Val Tyr Ser Asp Pro Pro Gly Leu Ala Pro Leu Pro
   20 25 30
   Glu Glu Glu Pro Leu Ile Ala Arg Ser Val Ala Lys Arg Arg Asn Glu
   35 40 45
   Phe Ile Thr Val Arg His Cys Ala Arg Ile Ala Leu Gly Glu Leu Gly
   50 55 60
   Leu Pro Pro Ala Pro Ile Leu Lys Gly Glu Lys Gly Glu Pro Arg Trp
   65 70 75 80
   Pro Asp Gly Val Val Gly Ser Leu Thr His Cys Thr Gly Tyr Arg Gly
   85 90 95
   Ala Val Val Gly Arg Thr Gly Ala Val Arg Ser Val Gly Ile Asp Ala
   100 105 110
   Glu Pro His Asp Val Leu Pro Asp Gly Val Leu Asn Ala Ile Ser Leu
   115 120 125
   Pro Ala Glu Arg Ser Glu Ile Pro Ser Ala Leu Pro Gly Asp Leu His
   130 135 140
   Trp Asp Arg Ile Leu Phe Cys Ala Lys Glu Ala Thr Tyr Lys Ala Trp
   145 150 155 160
   Phe Pro Leu Thr Arg Arg Trp Leu Gly Phe Glu Asp Ala Arg Ile Thr
   165 170 175
   Phe Glu Ala Asp His Pro Gly Ala Thr Thr Gly Gly Phe Val Ser Arg
   180 185 190
   Ile Leu Ile Asp Pro Ala Ala Leu Cys Gly Pro Pro Leu Thr Ala Leu
   195 200 205
   Ser Gly Arg Trp Ser Val Ala Arg Gly Leu Val Leu Thr Ala Ile Val
   210 215 220
   Leu
   225
<210> 9
   <211> 230
   <212> PRT
   <213> Corynebacterium diphtheriae
<220>
   <221> misc_feature
   <223> PptT
<400> 9
   Met Ile Ala Gly Asn Asn Ser Glu Tyr Ala Leu Asp Ser Arg Leu Phe
   1 5 10 15
   Pro Gln Ser Ala Arg Ser Thr Ala Leu Leu Val Pro Arg His Thr Pro
   20 25 30
   Asp Leu Ser Asn Phe Asn Arg Leu His Val Leu Glu Lys Ala Gln Val
   35 40 45
   Lys Asn Ala Val Ala Val Arg Arg Ala Glu Phe Gly Asp Ala Arg Trp
   50 55 60
   Cys Ala His Gln Ser Leu Arg Lys Leu Gly Leu Tyr Asp His Pro Ala
   65 70 75 80
   Ile Leu Arg Gly Glu Arg Gly Met Pro Leu Trp Pro Val Gly Ile Ala
   85 90 95
   Gly Ser Leu Thr His Thr Glu Gly Leu Arg Ala Ala Val Val Ala Pro
   100 105 110
   Thr Thr Glu Val Ala Ser Met Gly Ile Asp Ala Glu Ile Ala Glu Glu
   115 120 125
   Leu Pro Gly Gly Ile Leu Gly Ser Ile Ala Arg Pro Asn Glu Ile Ala
   130 135 140
   Met Leu Asp Asp Leu Arg Ala Arg Gly Leu Leu Phe Ala Asp Arg Leu
   145 150 155 160
   Leu Phe Cys Ala Lys Glu Ala Thr Tyr Lys Ala Trp Phe Pro Ile Thr
   165 170 175
   Gln Arg Trp Leu Asp Phe Asp Gln Ala Glu Ile Asp Ile Arg Ala Asp
   180 185 190
   Gly Thr Phe Ile Ser Tyr Leu Leu Ile Arg Pro Thr Pro Phe Pro Phe
   195 200 205
   Ile Glu Gly Lys Trp Ala Ile His Asp Gly Tyr Val Val Ala Thr Thr
   210 215 220
   Val Ile Pro Ala Met Gly
   225 230